# EUROPEAN PATENT APPLICATION

(11) **EP 3 754 595 A1**
(43) Date of publication of application: **23.12.2020**
(21) Application number: 18906635.0
(22) Date of filing: 16.02.2018
(51) Int. Cl.: G06T 7/00, C12M 1/34

(54) **CALCULATION DEVICE, CALCULATION PROGRAM, AND CALCULATION METHOD**

(71) Applicant: NIKON CORPORATION, Minato-ku Tokyo 108-6290 (JP)
(72) Inventor: HATTO Mao, Tokyo 108-6290 (JP); FURUTA Shinichi, Tokyo 108-6290 (JP); MASUTANI Mamiko, Tokyo 108-6290 (JP); HAYASHI Seri, Tokyo 108-6290 (JP)
(74) Representative: Hoffmann Eitle
(86) International application number: PCT/JP2018/005511
(87) International publication number: WO 2019/159326

(57) **Abstract**

A calculation device includes a reference image acquisition unit configured to acquire a plurality of reference images in which cells are imaged, a comparative image acquisition unit configured to acquire a comparative image in which comparative cells to be compared with the cells imaged in the plurality of reference images are imaged, and a calculation unit configured to calculate the difference between a feature quantity calculated using the plurality of reference images and a feature quantity calculated using the comparative image.

## Description

### [Technical Field]

The present invention relates to a calculation device, a calculation program, and a calculation method.

### [Background Art]

In the biological and medical sciences and the like, it is known that there is a correlation, for example, between a state of health, disease, or the like of an organism and a state of cells, intracellular organelles, or the like. Thus, the analysis of a correlation between these is one means for solving various issues in the biological and medical sciences and the like. A state of cells, cellular organelles, or the like is observed to know a state of cells, intracellular organelles, or the like. However, the observation of cells by the vision of an observer is significantly dependent on the subjectivity of the observer and it is difficult to obtain an objective analysis result. As technology for objectively analyzing cells, tissue slices, and the like, for example, technologies using image processing are known (see, for example, Patent Document 1). Conventionally, it is desirable to quantify differences between images of cells and tissue slices and the like as a method of objectively analyzing cells, tissue slices and the like.

### [Citation List]

### [Patent Literature]

[Patent Document 1]
United States Patent No. 9280698

### [Summary of Invention]

There is provided a calculation device including: a reference image acquisition unit configured to acquire a plurality of reference images in which cells are imaged; a comparative image acquisition unit configured to acquire a comparative image in which comparative cells to be compared with the cells imaged in the plurality of reference images are imaged; and a calculation unit configured to calculate the difference between a feature quantity calculated using the plurality of reference images and a feature quantity calculated using the comparative image.

According to a second aspect of the present invention, there is provided a calculation program for causing a computer to execute: a reference image acquisition step of acquiring a plurality of reference images in which cells are imaged; a comparative image acquisition step of acquiring a comparative image in which comparative cells to be compared with the cells imaged in the plurality of reference images are imaged; and a calculation step of calculating the difference between a feature quantity calculated using the plurality of reference images and a feature quantity calculated using the comparative image.

According to a third aspect of the present invention, there is provided a calculation method for executing: a reference image acquisition means for acquiring a plurality of reference images in which cells are imaged; a comparative image acquisition means for acquiring a comparative image in which comparative cells to be compared with the cells imaged in the plurality of reference images are imaged; and a calculation means for calculating the difference between a feature quantity calculated using the plurality of reference images and a feature quantity calculated using the comparative image.

### [Brief Description of Drawings]

Fig. 1 is a diagram showing an example of a configuration of a microscope observation system according to a first embodiment of the present invention.
Fig. 2 is a block diagram showing an example of a functional configuration of units provided in a calculation device according to the present embodiment.
Fig. 3 is a diagram showing an example of an arithmetic operation procedure of calculating a reference feature quantity in an arithmetic operation unit according to the present embodiment.
Fig. 4 is a diagram showing an example of a method of calculating a feature quantity using a neural network according to the present embodiment.
Fig. 5 is a flowchart showing an example of an arithmetic operation procedure of quantifying differences between a plurality of reference images and one comparative image in the arithmetic operation unit according to the present embodiment.
Fig. 6 is a diagram showing an example of a process of quantifying differences between a plurality of reference images and one comparative image according to the present embodiment.
Fig. 7 is a flowchart showing an example of an arithmetic operation procedure of the arithmetic operation unit for a plurality of comparative images according to the present embodiment.
Fig. 8 is a diagram showing an example of a process of quantifying differences between a plurality of reference images and a plurality of comparative images according to the present embodiment.
Fig. 9 is a block diagram showing an example of a functional configuration of units provided in a calculation device according to a second embodiment of the present invention.
Fig. 10 is a flowchart showing an example of an arithmetic operation procedure of an arithmetic operation unit for a plurality of comparative images according to the present embodiment.
Fig. 11 is a diagram showing an example of calculation of response proportions of a plurality of comparative images according to the present embodiment.
Fig. 12 is a diagram showing an example of a process of selecting a comparative image according to the present embodiment.
Fig. 13 is a diagram showing an example of a process of determining a position within a well according to the present embodiment.
Fig. 14 is a block diagram showing an example of a functional configuration of units provided in a calculation device according to a third embodiment of the present invention.
Fig. 15 is a flowchart showing an example of an arithmetic operation procedure of an arithmetic operation unit according to the present embodiment.
Fig. 16 is a diagram showing an example of differences between a plurality of reference images and a plurality of comparative images for each time series according to the present embodiment.
Fig. 17 is a diagram showing an example of differences between a plurality of reference images and a plurality of comparative images for each concentration of a compound according to the present embodiment.
Fig. 18 is a diagram showing an example of differences between a plurality of reference images and a plurality of comparative images for each type of compound according to the present embodiment.
Fig. 19 is a block diagram showing an example of a functional configuration of units provided in a calculation device according to a fourth embodiment of the present invention.
Fig. 20 is a flowchart showing an example of a procedure of calculating a reference feature quantity in a calculation unit of the present embodiment.
Fig. 21 is a flowchart showing an example of an arithmetic operation procedure of classifying target images into classes in an arithmetic operation unit according to the present embodiment.
Fig. 22 is a diagram showing an example of a target image classification process according to the present embodiment.
Fig. 23 is a block diagram showing an example of a functional configuration of units provided in a calculation device according to a fifth embodiment of the present invention.
Fig. 24 is a diagram showing an example of an arithmetic operation procedure of determining a culture state in an arithmetic operation unit 100d according to the present embodiment.
Fig. 25 is a diagram showing an example of a culture state determination process according to the present embodiment.
Fig. 26 is a diagram showing a modified example of the arithmetic operation procedure of the arithmetic operation unit according to the present embodiment.
Fig. 27 is a diagram showing an example of cross-sectional images of a spheroid according to the present embodiment.
Fig. 28 is a block diagram showing an example of a functional configuration of units provided in a calculation device according to a sixth embodiment of the present invention.
Fig. 29 is a diagram showing an example of an arithmetic operation procedure of selecting an image in an arithmetic operation unit 100e according to the present embodiment.
Fig. 30 is a diagram showing an example of a spheroid image according to the present embodiment.
Fig. 31 is a flowchart showing an example of an arithmetic operation procedure of calculating a reference representative feature quantity in the arithmetic operation unit according to the present embodiment.

### [Description of Embodiments]

### [First embodiment]

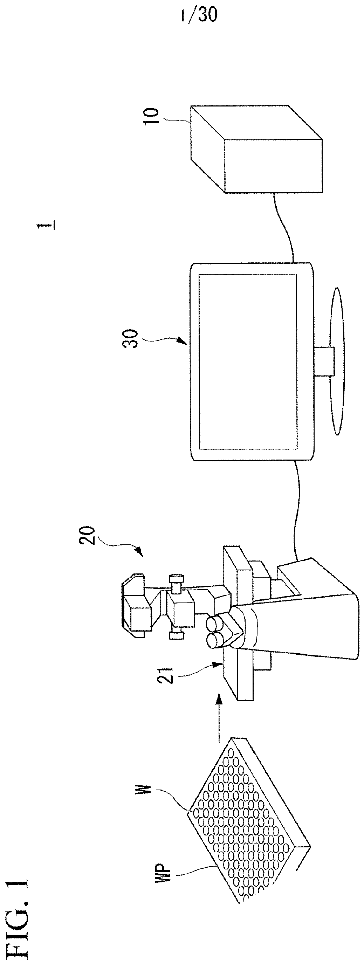

Hereinafter, embodiments of the present invention will be described with reference to the drawings. Fig. 1 is a diagram showing an example of a configuration of a microscope observation system 1 according to a first embodiment of the present invention.

The microscope observation system 1 performs image processing on an image obtained by imaging cells and the like. In the following description, the image obtained by imaging the cells and the like is also simply referred to as a cell image.

The microscope observation system 1 includes a calculation device 10, a microscope device 20, and a display unit 30.

The microscope device 20 is a biological microscope and includes an electromotive stage 21 and an imaging unit 22 (not shown). The electromotive stage 21 can arbitrarily move a position of an imaging object in a predetermined direction (e.g., a certain direction within a two-dimensional plane of a horizontal direction, a vertical direction, or an axis rotation direction).

The imaging unit 22 includes an imaging element such as a charge-coupled device (CCD) or a complementary MOS (CMOS) and is configured to image the imaging object on the electromotive stage 21. Also, the microscope device 20 may not include the electromotive stage 21 and the stage may be a stage that does not operate in the predetermined direction or a stage that manually operates in the predetermined direction.

More specifically, for example, the microscope device 20 has functions of a differential interference contrast microscope (DIC), a phase contrast microscope, a fluorescence microscope, a confocal microscope, a super-resolution microscope, a two-photon excitation fluorescence microscope, a light sheet microscope, a light field microscope, a holographic microscope, an optical interference tomography (OCT) device, and the like.

The microscope device 20 captures an image of a culture vessel placed on the electromotive stage 21. For example, this culture vessel is a well plate WP, a slide chamber, or the like. The microscope device 20 irradiates cells cultured inside a large number of wells W provided in the well plate WP with light and therefore images light transmitted through or reflected on the cells as a cell image. Thereby, the microscope device 20 can acquire an image such as a transmission DIC image, a phase contrast image, a dark field image, or a bright field image for the cells.

Further, the microscope device 20 captures an image of fluorescence emitted from a fluorescent substance as the cell image by irradiating the cells with excitation light. Further, the microscope device 20 captures an image of emitted light or phosphorescence from a light-emitting substance in the cells as the cell image.

In the present embodiment, cells are stained alive and time-lapse photographing is performed to acquire a cell change image after cell stimulation. In the present embodiment, a cell image is acquired by expressing a fluorescent fusion protein or staining the cells with a chemical reagent or the like while the cells are alive. In still another embodiment, cells are fixed and stained to acquire a cell image. Metabolism ceases in fixed cells. Therefore, when an intracellular change over time is observed in fixed cells after a stimulus is applied to the cells, it is necessary to provide a plurality of cell culture vessels seeded with cells. For example, when there are a plurality of wells inside a plate, the wells may be used as cell culture vessels. In this case, fixed cells with different elapsed time periods for a stimulus may be provided for each well in the plate. Of course, fixed cells with different elapsed time periods for a stimulus may be provided for each plate.

Also, the microscope device 20 may image emitted light or fluorescence from a chromogenic substance itself incorporated in a biological substance or emitted light or fluorescence generated through binding of a substance having a chromophore with the biological substance as the above-described cell image. Thereby, the microscope observation system 1 can acquire a fluorescent image, a confocal image, a super-resolution image, and a two-photon excitation fluorescence microscope image.

Also, a method of acquiring a cell image is not limited to an optical microscope. For example, a method of acquiring a cell image may be an electron microscope, i.e., a type of cell image may be appropriately selected.

In the present embodiment, the cells include, for example, cells such as primary cultured cells, subculture cells, and tissue sections. In order to observe cells, an observed sample may be an aggregate of cells, a tissue sample, an organ, or an individual (such as an animal) and may be acquired as an image including cells. Also, the state of the cells is not particularly limited and may be a living state or may be a fixed state. Of course, information of the living state and information of the fixed state may be combined.

Also, cells may be treated with a chemiluminescent or fluorescent protein (for example, a chemiluminescent or fluorescent protein expressed from an introduced gene (green fluorescent protein (GFP) or the like)) and observed. Alternatively, the cells may be observed using immunostaining or staining with a chemical reagent. The cells may be observed by combining these. For example, it is also possible to select the photoprotein to be used in accordance with a type for discriminating an intracellular nuclear structure (e.g., a Golgi body or the like).

The well plate WP includes one or more wells W. As one example, the well plate WP includes 96 (=8×12) wells W as shown in Fig. 1. The number of wells W provided in the well plate WP is not limited to this and may be 48 (=6×8), 24 (=6×4), 12 (=3×4), 6 (=2×3), 384 (=12×32), or 1536 (=32×48). The cells are cultured within the wells W under specific experimental conditions. The specific experimental conditions include temperature, humidity, culture period, elapsed time period after a stimulus is applied, type and strength of the applied stimulus, concentration, amount, presence or absence of a stimulus, induction of biological features, and the like. The stimulus is, for example, a physical stimulus of electricity, sound waves, magnetism, light, or the like or a chemical stimulus obtained by administering a substance, a drug or the like. Also, the biological features are features that represent the step of differentiation of cells, morphology, the number of cells, the behavior of molecules in the cells, the morphology and behavior of organelles, the behavior of an intranuclear structure, the behavior of DNA molecules, and the like.

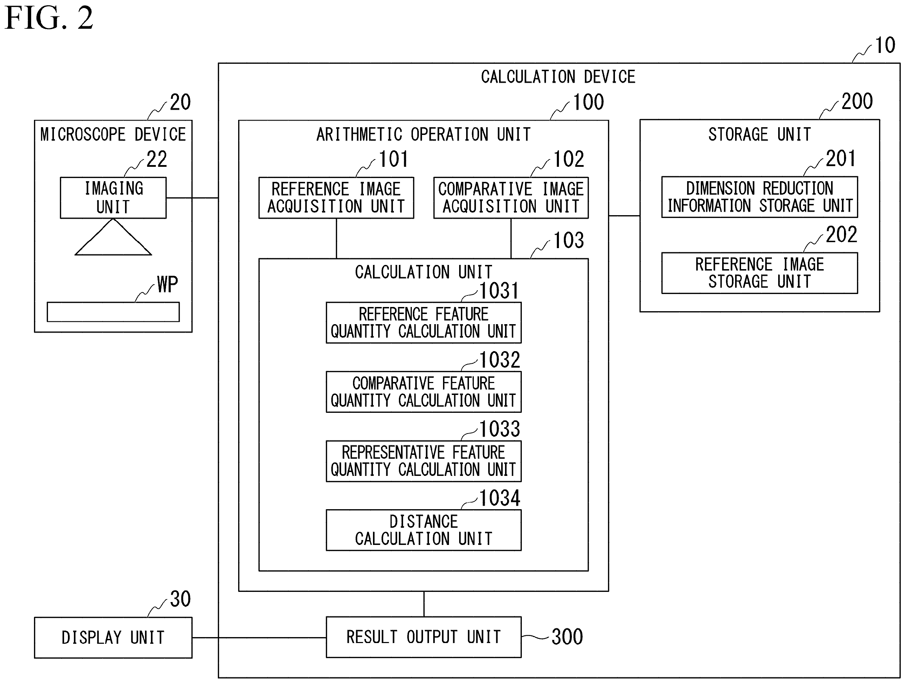

Fig. 2 is a block diagram showing one example of a functional configuration of units provided in the calculation device 10 according to the present embodiment. The calculation device 10 is a computer device that analyzes an image acquired by the microscope device 20.

The calculation device 10 includes an arithmetic operation unit 100, a storage unit 200, and a result output unit 300.

Also, the images on which the calculation device 10 performs image processing are not limited to the images captured by the microscope device 20, and may be, for example, images pre-stored in the storage unit 200 provided in the calculation device 10, or may be images pre-stored in an external storage device (not shown).

The arithmetic operation unit 100 functions when a program stored in the storage unit 200 is executed by a processor. Some or all of these functional units of the arithmetic operation unit 100 may include hardware such as a large-scale integration (LSI) circuit or an application specific integrated circuit (ASIC). The arithmetic operation unit 100 includes a reference image acquisition unit 101, a comparative image acquisition unit 102, and a calculation unit 103.

The reference image acquisition unit 101 acquires a plurality of reference images stored in the reference image storage unit 202 of the storage unit 200 and supplies the plurality of reference images that have been acquired to the calculation unit 103. Here, the reference image is an image in which cells are imaged and is an image which is used for comparison with a comparative image. The plurality of reference images are a plurality of images of cells cultured under the same experimental conditions. It is preferable that the plurality of reference images be images of cells to which no stimulus is applied.

The comparative image acquisition unit 102 acquires one or more cell images captured by the imaging unit 22 as one or more comparative images and supplies the one or more comparative images that have been acquired to the calculation unit 103. Here, the comparative image is an image in which comparative cells, which are a target to be compared with the cells imaged in the plurality of reference images, are imaged. The comparative image is, for example, an image of cells when a predetermined time period has elapsed after the application of the stimulus. It is preferable that experimental conditions in which the cells imaged in the comparative image are cultured (experimental conditions of a stimulus and the like other than target items desired to be compared and examined) be the same as experimental conditions in which the cells imaged in the reference image are cultured (experimental conditions of a stimulus and the like other than target items desired to be compared and examined). It is preferable that the experimental conditions for the cells imaged in the comparative image be the same as the experimental conditions for the cells imaged in the reference image, except for items desired to be compared and examined. For example, when the stimulus has been changed between the reference image and the comparative image, it is preferable that experimental conditions such as culture conditions for cells imaged in the reference image be the same as experimental conditions such as culture conditions for cells imaged in the comparative image, except for the stimulus conditions. As the stimulus conditions, for example, the reference image is a cell image of a condition in which no stimulus is applied to cells and the comparative image is a cell image of a condition in which a stimulus is applied to cells. Of course, as the stimulus condition, for example, a type of chemical liquid applied to cells as a stimulus is different.

Also, hereinafter, a plurality of reference images may be referred to as a reference image group. Also, one or more comparative images may be referred to as a comparative image group.

The calculation unit 103 calculates the difference between a feature quantity calculated on the basis of a plurality of reference images and a feature quantity calculated on the basis of one comparative image. The calculation unit 103 sequentially sets one or more comparative images supplied to the calculation unit 103 by the comparative image acquisition unit 102 as a target of the calculation process one by one. In the present embodiment, the feature quantity calculated on the basis of the reference image is a value to which a cell image feature included in the reference image is applied. The cell image feature includes, for example, luminance of the cell image, a cell area in the image, dispersion of the luminance of the cell image in the image, a shape, and the like. That is, the feature quantity is a feature derived from information acquired from the imaged cell image. As described above, the feature quantity includes the image feature quantity regarding the cells.

In the present embodiment, the feature quantity calculated using the reference image includes a plurality of feature quantities. In the present embodiment, the feature quantity calculated on the basis of the reference image includes a plurality of types of feature quantities. Here, for example, the plurality of types of feature quantities are feature quantities representing a plurality of features extracted from the cell image, such as the luminance of the cell image and the cell area in the image. As described above, the feature quantity calculated on the basis of the plurality of reference images includes a plurality of feature quantities.

Also, in a feature quantity calculation method to be described below, the feature quantity extracted from the cell image may be predetermined. For example, at least the cell area in the image may be determined to be extracted and the feature quantity may be calculated so that the calculated feature quantity becomes a feature quantity to which at least the cell area in the image is applied. Also, the cell image includes images of a plurality of types of living tissues having different sizes such as genes, proteins, and organelles. Therefore, elements constituting the cells included in the cell image are determined. The feature quantity extracted from the cell image is calculated for each determination result of the elements constituting the cells. Here, the constituent elements of the cells include cell nuclei, lysosomes, Golgi bodies, organelles such as mitochondria, and proteins constituting organelles.

Also, the feature quantity calculated using the comparative image is similar to the feature quantity calculated using the reference image. Therefore, the feature quantity calculated on the basis of the plurality of comparative images includes a plurality of feature quantities.

The calculation unit 103 includes a reference feature quantity calculation unit 1031, a comparative feature quantity calculation unit 1032, a representative feature quantity calculation unit 1033, and a distance calculation unit 1034.

The reference feature quantity calculation unit 1031 calculates feature quantities of the reference images included in the reference image group supplied by the reference image acquisition unit 101 as a plurality of reference feature quantities. The reference feature quantity calculation unit 1031 supplies the calculated plurality of reference feature quantities to the representative feature quantity calculation unit 1033.

The comparative feature quantity calculation unit 1032 calculates a feature quantity of the comparative image included in the one or more comparative images supplied by the comparative image acquisition unit 102 as a comparative feature quantity. The comparative feature quantity calculation unit 1032 supplies the calculated comparative feature quantity to the distance calculation unit 1034.

The reference feature quantity calculation unit 1031 calculates a reference feature quantity by dimensionally reducing the reference image. The comparative feature quantity calculation unit 1032 calculates a comparative feature quantity by dimensionally reducing the comparative image. A dimension reduction method in which the reference feature quantity calculation unit 1031 calculates the reference feature quantity is the same as a dimension reduction method in which the comparative feature quantity calculation unit 1032 calculates the comparative feature quantity, except that a target image whose feature quantity is calculated is different. Details of this dimension reduction will be described below.

The representative feature quantity calculation unit 1033 calculates a reference representative feature quantity on the basis of the plurality of reference feature quantities supplied by the reference feature quantity calculation unit 1031. Here, the reference representative feature quantity is a representative value of a distribution of the plurality of reference feature quantities. The representative feature quantity calculation unit 1033 supplies the calculated reference representative feature quantity to the distance calculation unit 1034.

The distance calculation unit 1034 calculates a distance between the reference representative feature quantity and the comparative feature quantity using the reference representative feature quantity supplied by the representative feature quantity calculation unit 1033 and the comparative feature quantity supplied by the comparative feature quantity calculation unit 1032. The calculated distance represents the difference between the plurality of reference images and the comparative image. Because a representative value calculated from the reference feature quantities of the plurality of reference images is used in the present embodiment, the difference between the image corresponding to the reference representative feature quantity and the comparative image is represented. Here, a size of the difference between the plurality of reference images and the comparative image represents the size of the difference between the state of the cells imaged in the plurality of reference images and the state of the cells imaged in the comparative image. Therefore, when the difference between the plurality of reference images and the comparative image becomes large, the state of the cells imaged in the plurality of reference images and the state of the cells imaged in the comparative image become more different. Here, the difference in the state of the cells is, for example, the difference in the response of the cells to the stimulus. The distance calculation unit 1034 supplies the calculated distance to the result output unit 300.

Here, the distance calculated by the distance calculation unit 1034 is, for example, a Euclidean distance between one or more values representing the feature quantity. In this case, the difference calculated by the calculation unit 103 is a value calculated on the basis of the difference between corresponding values among one or more values representing the feature quantity calculated using the plurality of reference images and one or more values representing the feature quantity calculated using the comparative image. That is, the difference calculated by the calculation unit 103 is a value calculated on the basis of a relationship between corresponding values among one or more values representing the feature quantity calculated using the plurality of reference images and one or more values representing the feature quantity calculated using the comparative image.

Also, the distance calculated by the distance calculation unit 1034 may be a distance other than a Euclidean distance. The distance calculated by the distance calculation unit 1034 may be, for example, a standard Euclidean distance, a Mahalanobis distance, a Manhattan distance, a Chebyshev distance, a Minkowski distance, a degree of cosine similarity, or a Pearson product moment correlation.

Also, the distance calculated by the distance calculation unit 1034 may be a distance to which an outer product (a wedge product) is applied. The distance to which the outer product (the wedge product) is applied is calculated as follows. First, two-dimensional values are extracted from an N-dimensional vector A and an N-dimensional vector B and a two-dimensional vector a and a two-dimensional vector b are created. Next, an area of a triangle or a parallelogram of which two sides are the two-dimensional vector a and the two-dimensional vector b is calculated. The above-described operation is iterated a number of times equal to the number of cases in which two dimensions are selected from N dimensions and the above-described area is calculated with respect to a combination of _{N}C₂ types of dimensions from the N-dimensional vector A and the N-dimensional vector B. Here, the number of cases in which two dimensions are selected from N dimensions is represented by _{N}C₂. Finally, the calculated representative value of the area (a sum, an average or median value, or the like) is set as the distance between the N-dimensional vector A and the N-dimensional vector B.

The storage unit 200 includes a dimension reduction information storage unit 201 and a reference image storage unit 202. The dimension reduction information storage unit 201 stores information representing a dimension reduction procedure that is used when the reference feature quantity calculation unit 1031 and the comparative feature quantity calculation unit 1032 calculate the feature quantity of the cell image. Information representing the dimension reduction procedure will be described below. The reference image storage unit 202 stores a reference image group.

The result output unit 300 outputs a distance supplied by the calculation unit 103 to the display unit 30. Also, the result output unit 300 may output the distance supplied by the calculation unit 103 to an output device other than the display unit 30, a storage device, or the like.

The display unit 30 displays the distance output by the result output unit 300.

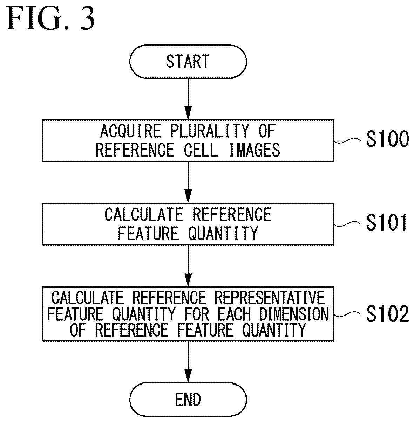

Fig. 3 is a flowchart showing an example of an arithmetic operation procedure of calculating a reference feature quantity in the arithmetic operation unit 100 according to the present embodiment. Also, the arithmetic operation procedure shown here is an example and the arithmetic operation procedure may be omitted or an arithmetic operation procedure may be added.

The reference image acquisition unit 101 acquires a reference image group S1 stored in the reference image storage unit 202 (step S100). The reference image acquisition unit 101 supplies the acquired reference image group S1 to the reference feature quantity calculation unit 1031.

The reference feature quantity calculation unit 1031 calculates a reference feature quantity of each of reference images included in the reference image group S1 supplied by the reference image acquisition unit 101 (step S101). Here, the reference feature quantity calculation unit 1031 calculates a reference feature quantity by dimensionally reducing the reference image. The reference feature quantity calculation unit 1031 supplies a plurality of reference feature quantities that have been calculated to the representative feature quantity calculation unit 1033.

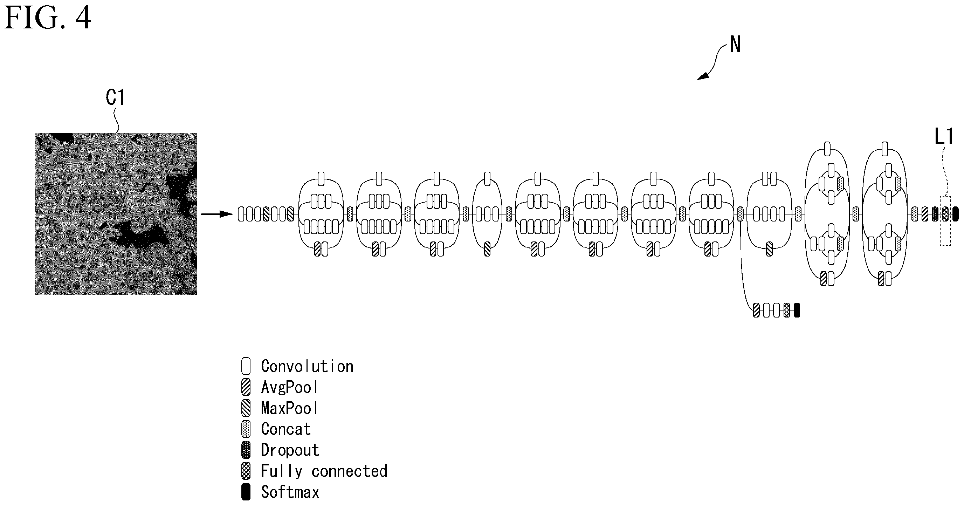

In the present embodiment, the reference feature quantity calculation unit 1031 calculates the reference feature quantity using a multilayer neural network. The multilayer neural network is a neural network that includes one or more intermediate layers. Here, a method of calculating the reference feature quantity in the reference feature quantity calculation unit 1031 will be described with reference to Fig. 4.

Fig. 4 is a diagram showing an example of a feature quantity calculation method using the neural network according to the present embodiment.

The neural network N includes a plurality of layers including an input layer, one or more intermediate layers, and an output layer. If an input image is input to the input layer, the neural network N transfers information representing that a node of each layer has assigned a predetermined weight to each node the next layer and subsequent layers. Here, the fact that the input image is input to the input layer means that luminance values of pixels of the input image are input to an input layer. Therefore, the number of nodes constituting the input layer of the neural network N is equal to the number of pixels constituting the input image. The neural network N classifies input images into a predetermined number of categories on the basis of the information transferred to the output layer. The number of nodes of the output layer is equal to the number of categories into which the images are classified by the neural network N. In the present embodiment, the number of nodes of the output layer of the neural network N is 1000 as an example.

Also, the neural network N may be an auto encoder in which the number of nodes of the input layer is equal to the number of nodes of the output layer. Because the number of nodes of the intermediate layer is generally smaller than the number of nodes of the input layer in the auto encoder, a feature of the input image can be efficiently extracted in the intermediate layer. Further, the neural network N may be an auto encoder in which the number of input layers is different from the number of output layers.

The neural network N is, for example, a convolutional neural network (CNN), and has a convolutional layer, a pooling layer, a connection layer, and a dropout layer as intermediate layers. In the intermediate layer, a feature of the input image is extracted. As the feature of the input image extracted in the intermediate layer, a higher-order feature is extracted when the intermediate layer is closer to the output layer side. For example, in the intermediate layer close to the input layer, a simple pattern feature such as an edge of the input image is extracted as the feature of the input image. On the other hand, in the intermediate layer close to the output layer, a feature of a complicated pattern is extracted as the feature of the input image. The feature of the input image extracted in the intermediate layer is represented by a set of values output from the nodes constituting the intermediate layer.

In the present embodiment, a set of values output from nodes of an intermediate layer L1 adjacent to the output layer of the neural network N is calculated as a feature quantity of a cell image C1.

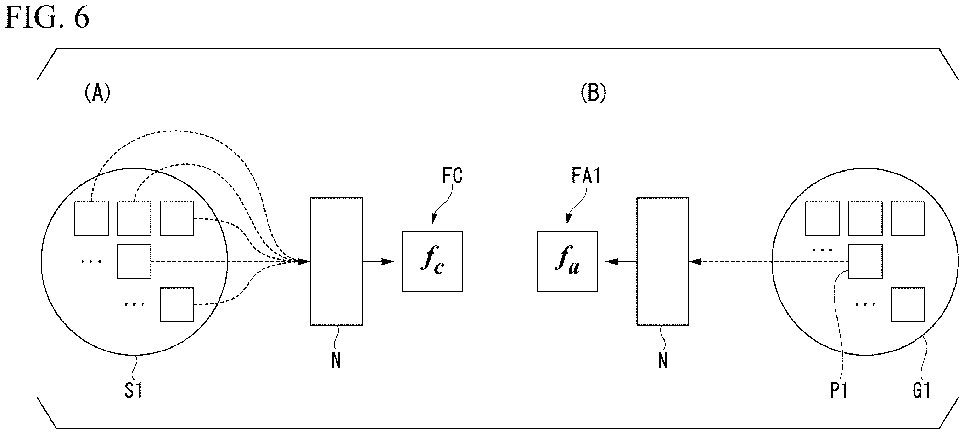

Returning to Fig. 3, a description of an arithmetic operation procedure of calculating a reference feature quantity in the arithmetic operation unit 100 will be continued. Here, the arithmetic operation procedure of calculating a reference feature quantity in the arithmetic operation unit 100 will be described with reference to Fig. 6.

Fig. 6 is a diagram showing an example of a process of quantifying the difference between a plurality of reference images and one comparative image. In Fig. 6(A), the reference feature quantity calculation unit 1031 inputs reference images included in a reference image group S1 to the neural network N. Here, the reference feature quantity calculation unit 1031 uses the neural network N stored in the dimension reduction information storage unit 201. The neural network N stored in the dimension reduction information storage unit 201 is, for example, a neural network learned with 12 million pieces of learning data. A learning image included in the learning data may be a cell image or a general image other than the cell image. In this manner, the reference feature quantity calculation unit 1031 uses a feature quantity calculated by dimensionally reducing the input image by means of the neural network including an input layer, one or more intermediate layers, and an output layer and configured to transfer information representing that a node of each layer has assigned a predetermined weight to each node of the next layer and subsequent layers.

For example, the reference feature quantity calculation unit 1031 calculates a set of values output from nodes of the intermediate layer L1 adjacent to the final output layer of the neural network N as the reference feature quantity of the reference image. Here, the number of dimensions of the reference feature quantity is equal to the number of nodes of the intermediate layer L1 of the neural network N. In the present embodiment, the number of nodes of the input layer is 65536 as an example and the number of nodes of the intermediate layer L1 is 2048 as an example. That is, luminance information of 65536 dimensions of the input image is dimensionally reduced to 2048 dimensions of the reference feature quantity. The number of dimensions of the reference feature quantity is not limited to 2048. For example, the number of dimensions of the reference feature quantity is preferably 50 or more.

The representative feature quantity calculation unit 1033 calculates a reference representative feature quantity FC for each dimension of the reference feature quantity from a plurality of reference feature quantities supplied by the reference feature quantity calculation unit 1031 (step S102). The representative feature quantity calculation unit 1033 generates a distribution of a plurality of reference feature quantities for each dimension of the reference feature quantity and calculates each representative value of the generated distribution as the reference representative feature quantity FC. The representative feature quantity calculation unit 1033 generates a distribution regarding the reference feature quantity from the values of the plurality of reference feature quantities and calculates the reference representative feature quantity FC using a representative value of the generated distribution. Here, the representative value of the distribution is, for example, an average value of the distribution. Also, a median value or a mode value may be used as the representative value of the distribution. The representative feature quantity calculation unit 1033 supplies the calculated reference representative feature quantity FC to the distance calculation unit 1034.

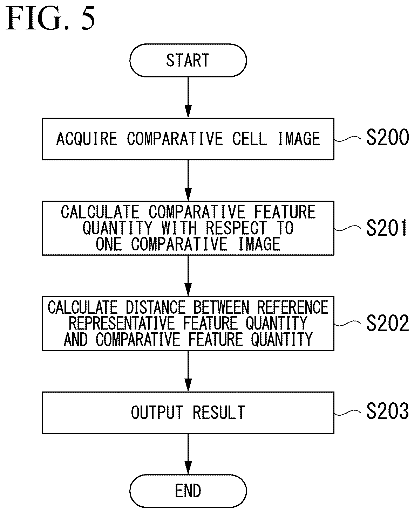

Fig. 5 is a flowchart showing an example of an arithmetic operation procedure of quantifying the difference between a plurality of reference images and one comparative image in the arithmetic operation unit 100 according to the present embodiment.

The comparative image acquisition unit 102 acquires a cell image captured by the imaging unit 22 as a comparative image (step S200). The comparative image acquisition unit 102 supplies one comparative image that has been acquired to the comparative feature quantity calculation unit 1032.

The comparative feature quantity calculation unit 1032 calculates a comparative feature quantity of one comparative image P1 of one or more comparative image groups G1 supplied by the comparative image acquisition unit 102 (step S201). The comparative feature quantity calculation unit 1032 calculates a comparative feature quantity FA1 of the comparative image P1 using a neural network as a dimension reduction technique.

A process of quantifying the difference between a plurality of reference images and one comparative image P1 will be described with reference to Fig. 6(B). The comparative feature quantity calculation unit 1032 inputs the comparative image P1 included in the comparative image group G1 to the neural network N. Here, the comparative feature quantity calculation unit 1032 calculates a feature quantity using the neural network N stored in the dimension reduction information storage unit 201.

The comparative feature quantity FA1 calculated by the comparative feature quantity calculation unit 1032 is a set of values output from nodes of the intermediate layer L1 when the comparative image P1 has been input to the neural network N. The number of dimensions of the comparative feature quantity FA1 is equal to the number of nodes of the intermediate layer L1 of the neural network N. The comparative feature quantity calculation unit 1032 supplies the calculated comparative feature quantity FA1 to the distance calculation unit 1034.

Returning to Fig. 5, a description of the arithmetic operation procedure of the arithmetic operation unit 100 will be continued.

The distance calculation unit 1034 calculates a distance between a reference representative feature quantity FC supplied by the representative feature quantity calculation unit 1033 and a comparative feature quantity FA1 supplied by the comparative feature quantity calculation unit 1032 (step S202). Here, the reference representative feature quantity is a feature quantity calculated on the basis of a plurality of reference images. Also, the comparative feature quantity is a feature quantity calculated on the basis of the comparative image. Therefore, the calculation unit 103 calculates a difference using a reference representative feature quantity calculated by the representative feature quantity calculation unit 1033 and a feature quantity obtained by dimensionally reducing the comparative image calculated by the comparative feature quantity calculation unit 1032 from a feature quantity obtained by dimensionally reducing the reference image calculated by the reference feature quantity calculation unit 1031. That is, the calculation unit 103 uses a reference image acquired by the reference image acquisition unit 101, a comparative image acquired by the comparative image acquisition unit 102, and feature quantities obtained by dimensionally reducing the reference image and the comparative image to calculate the difference between a feature quantity calculated using a plurality of reference images and a feature quantity calculated using a comparative image. Here, the difference is the difference between a representative feature quantity calculated on the basis of the plurality of reference images and the feature quantity calculated on the basis of the comparative image. The distance calculation unit 1034 supplies the calculated distance to the result output unit 300.

The result output unit 300 outputs a result by causing the display unit 30 to display a distance supplied by the distance calculation unit 1034 (step S203).

Also, although a case in which the reference feature quantity calculation unit 1031 and the comparative feature quantity calculation unit 1032 calculate the value output from each node of the intermediate layer L1 of the neural network N as the feature quantity of the input cell image has been described in the present embodiment, the reference feature quantity calculation unit 1031 and the comparative feature quantity calculation unit 1032 may calculate a value output from each node of the intermediate layer of the neural network N other than the intermediate layer L1 as the feature quantity of the input cell image. That is, the calculation unit 103 may use an output of any one of the intermediate layers constituting the neural network N.

Also, the calculation unit 103 may use a value output from the output layer of the neural network N as the feature quantity. That is, the calculation unit 103 may use a deep learning determination result as the feature quantity.

Also, the neural network N may include only an input layer and an output layer without an intermediate layer.

Also, although a case in which a luminance value of a cell image is input as the input of the neural network N has been described in the present embodiment, a value other than the luminance value of the cell image may be input to the neural network N. For example, the average luminance or area of cells obtained through image analysis may be input as the feature quantity. Also, an HOG feature quantity of the input image, a filtered feature quantity, an SIFT feature quantity, an SURF feature quantity, and the like may be input to the neural network N.

Also, although a case in which a neural network is used as the dimension reduction method has been described in the present embodiment, a dimension reduction method other than the neural network may be used. For example, a technique such as principal component analysis, random projection, linear discriminant analysis, a multidimensional scaling method, random forest, isometric mapping, locally linear embedding, or spectral embedding may be used.

As described above, the calculation device 10 of the present embodiment includes the reference image acquisition unit 101, the comparative image acquisition unit 102, and the calculation unit 103. The reference image acquisition unit 101 acquires a plurality of reference images that are a plurality of images in which cells are imaged. The comparative image acquisition unit 102 acquires a comparative image that is an image in which comparative cells, which are a target to be compared with the cells imaged in the plurality of reference images, are imaged. The calculation unit 103 calculates the difference between the feature quantity calculated using the plurality of reference images and the feature quantity calculated using the comparative image.

According to this configuration, it is possible to stably quantify the difference, for example, as compared with a case in which there is one reference image, because the calculation device 10 can quantify the difference between a plurality of reference images and a comparative image. For example, a case in which the feature quantity is calculated on the basis of one reference image is a case in which an image does not represent typical cells that are cells under the experimental conditions with respect to the cells imaged in the reference image. For example, a plurality of cells within the culture vessel where the reference image is captured may not be uniform due to a unique difference in the cells and a variation in the experimental conditions for the cells. Variations in the experimental conditions are a variation in a cell staining step and a variation in a cell culturing step. For example, the degree of staining differs according to cells and a feature quantity representing cells such as luminance of a protein of a captured cell image may be different when the cells have been imaged as a result. Therefore, in the present embodiment, it is possible to calculate a feature quantity representing an average of a plurality of cells within a culture vessel where a reference image is captured because a feature quantity calculated on the basis of a plurality of reference images is used and a plurality of reference images are used as compared with a case in which a feature quantity calculated on the basis of one reference is used.

Also, the feature quantity calculated on the basis of the plurality of reference images includes a plurality of feature quantities. According to this configuration, the calculation device 10 can quantify the difference between a plurality of reference images and a plurality of comparative images, for example, using a feature quantity to which luminance of the cell image, a cell area in the image, and the like are applied.

Also, the calculation unit 103 calculates the difference between a reference image group and a comparative image using the reference image acquired by the reference image acquisition unit 101, the comparative image acquired by the comparative image acquisition unit 102, and a feature quantity obtained by dimensionally reducing the reference image and the comparative image.

According to this configuration, it is possible to stably perform quantization as compared with a case of general calculation of an image feature quantity because the calculation device 10 can quantify a difference without using an extraction result of cells or the like that was unstable in the conventional method. Also, the calculation unit 103 can dimensionally reduce a reference image acquired by the reference image acquisition unit 101. In the present embodiment, a dimension of an image in which cells are imaged is a value of a pixel constituting an image in which cells are imaged. For example, when an image in which cells are imaged has 200 pixels in the vertical direction and 200 pixels in the horizontal direction, there are values of 40000 pixels in the captured cell image. Therefore, the number of dimensions of the image in which the cells are imaged is 40000. Here, the number of dimensions of an image is the number of elements constituting the image. That is, because the image of the imaged cells is represented using 40000 values, each of the 40000 values is an element constituting the image. In the present embodiment, a dimension-reduced value is calculated from 40000 dimensions. The dimension-reduced value is a 2048-dimensional value. Although the image in which the cells are imaged is represented using values of 40000 pixels, it also becomes possible to represent an image in which cells are imaged in a 2048-dimensional value obtained through dimension reduction. Also, in the present embodiment, the values of the pixels constituting the image are represented in 256 gradations. Of course, the values of the pixels constituting the image are not limited to 256 gradations.

Also, the calculation unit 103 calculates a difference using a dimension-reduced feature quantity through the neural network N including an input layer, one or more intermediate layers, and an output layer and configured to transfer information representing that a node of each layer has assigned a predetermined weight to each node of the next layer.

According to this configuration, it is possible to perform quantization to which a feature derived from a cell image is applied because the calculation device 10 can quantify the difference between cell images with a dimension reduction technique using an optimized neural network according to learning. Further, it is possible to change a calculated distance in accordance with cells imaged in an image and a degree of similarity in the quantification. In the present embodiment, it is possible to shorten a distance between cells having a high degree of similarity of an image and lengthen a distance between cells having a low degree of similarity of an image. Also, in the present embodiment, it is possible to obtain a dimension-reduced feature quantity without impairing a cell feature derived from an image to be used by performing dimension reduction using the neural network.

Also, the calculation unit 103 uses an output of any one of the intermediate layers constituting the neural network N. According to this configuration, it is possible to quantify the difference between images of cells cultured under different experimental conditions using feature quantities of cell images because the calculation device 10 can use a feature quantity of the intermediate layer before aggregation as a determination result output from the final output layer of the neural network N.

Also, the difference calculated by the calculation unit 103 is a value calculated on the basis of a relationship between corresponding values among one or more values representing a feature quantity calculated using a plurality of reference images and one or more values representing a feature quantity calculated using a comparative image. According to this configuration, the calculation device 10 can quantify the difference between images of cells cultured under different experimental conditions using a distance such as a Euclidean distance.

Also, the feature quantity of the present embodiment includes an image feature quantity regarding cells. According to this configuration, the calculation device 10 can quantify the difference between a plurality of reference images and a comparative image on the basis of a feature derived from information acquired from an imaged cell image.

### [Modified example of first embodiment]

In the above-described embodiment, a case in which the calculation device 10 calculates the difference between a feature quantity calculated on the basis of a plurality of reference images and a feature quantity calculated on the basis of one comparative image has been described. Here, a case in which there are a plurality of comparative images, i.e., a case in which the calculation device 10 calculates the difference between a feature quantity calculated on the basis of a plurality of reference images and a feature quantity calculated on the basis of the plurality of comparative images, will be described as a modified example. Hereinafter, parts different from those of the above-described embodiment will be mainly described.

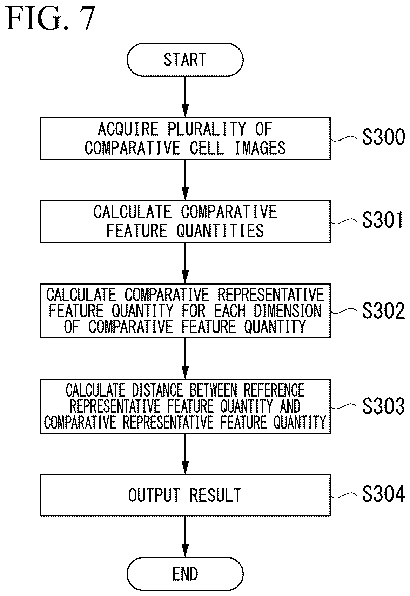

Fig. 7 is a flowchart showing an example of an arithmetic operation procedure of an arithmetic operation unit for a plurality of comparative images.

The comparative image acquisition unit 102 acquires a plurality of cell images captured by the imaging unit 22 as a plurality of comparative images (step S300). The comparative image acquisition unit 102 supplies the comparative feature quantity calculation unit 1032 with a plurality of comparative images that have been acquired as a comparative image group G1.

The comparative feature quantity calculation unit 1032 calculates a comparative feature quantity of each of comparative images P11 to P19 included in the comparative image group G1 supplied by the comparative image acquisition unit 102 (step S301).

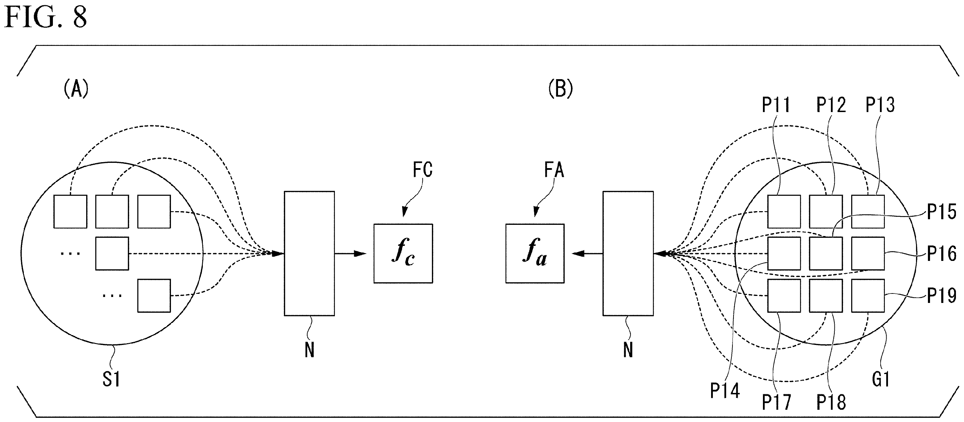

Here, a process of quantifying the difference between a plurality of reference images and a plurality of comparative images will be described with reference to Fig. 8(B).

Fig. 8 is a diagram showing an example of a process of quantifying the difference between a plurality of reference images and a plurality of comparative images according to the present embodiment. The comparative feature quantity calculation unit 1032 inputs the comparative images P11 to P19 included in the comparative image group G1 to the neural network N. The comparative feature quantity calculation unit 1032 calculates a comparative feature quantity for each of the comparative images P11 to P19 using the neural network as a dimension reduction technique.

Returning to Fig. 7, a description of an arithmetic operation procedure of the arithmetic operation unit for the plurality of comparative images will be continued.

The comparative feature quantity calculation unit 1032 supplies a plurality of comparative feature quantities that have been calculated to the representative feature quantity calculation unit 1033.

The representative feature quantity calculation unit 1033 calculates a comparative representative feature quantity FA for each dimension of the comparative feature quantity from the plurality of comparative feature quantities supplied by the comparative feature quantity calculation unit 1032 (step S302). Here, the comparative representative feature quantity FA is a representative value of a plurality of comparative feature quantities. The representative feature quantity calculation unit 1033 supplies the calculated comparative representative feature quantity FA to the distance calculation unit 1034.

The distance calculation unit 1034 calculates a distance between a reference representative feature quantity FC supplied by the representative feature quantity calculation unit 1033 and the comparative representative feature quantity FA supplied by the representative feature quantity calculation unit 1033 as the difference between a plurality of reference images and a plurality of comparative images (step S303). That is, the calculation unit 103 calculates the difference between a feature quantity calculated using the plurality of reference images and a feature quantity calculated using the plurality of comparative images.

The result output unit 300 outputs a result by causing the display unit 30 to display a distance supplied by the distance calculation unit 1034 (step S304).

As described above, in the present modified example, the comparative feature quantity calculation unit 1032 acquires the plurality of comparative images. The calculation unit 103 calculates the difference between the feature quantity calculated on the basis of the plurality of reference images and the feature quantity calculated on the basis of the plurality of comparative images.

According to this configuration, it is possible to calculate the difference between a plurality of reference images and a plurality of comparative images using a representative value of a feature quantity calculated from the plurality of reference images and a representative value of a feature quantity calculated from the plurality of comparative images. Therefore, the difference between the plurality of reference images and the plurality of comparative images can be represented by the difference between the representative values. For example, it is possible to curb an influence on quantification due to differences from the reference images by quantifying the difference between the plurality of reference images and the plurality of reference images using the representative values thereof.

Also, the feature quantity calculated on the basis of the plurality of comparative images includes a plurality of feature quantities. According to this configuration, for example, the calculation device 10 can quantify the difference between a plurality of reference images and a plurality of comparative images using a feature quantity to which luminance of a cell image, a cell area in an image, or the like is applied.

### [Second embodiment]

Hereinafter, a second embodiment of the present invention will be described with reference to the drawings.

In the above-described first embodiment, a case in which a calculation device quantifies the difference between a plurality of reference images and a comparative image(s) has been described. In the present embodiment, a case in which a calculation device selects a comparative image in which the difference between a quantified reference image and a comparative image is larger than a predetermined value will be described.

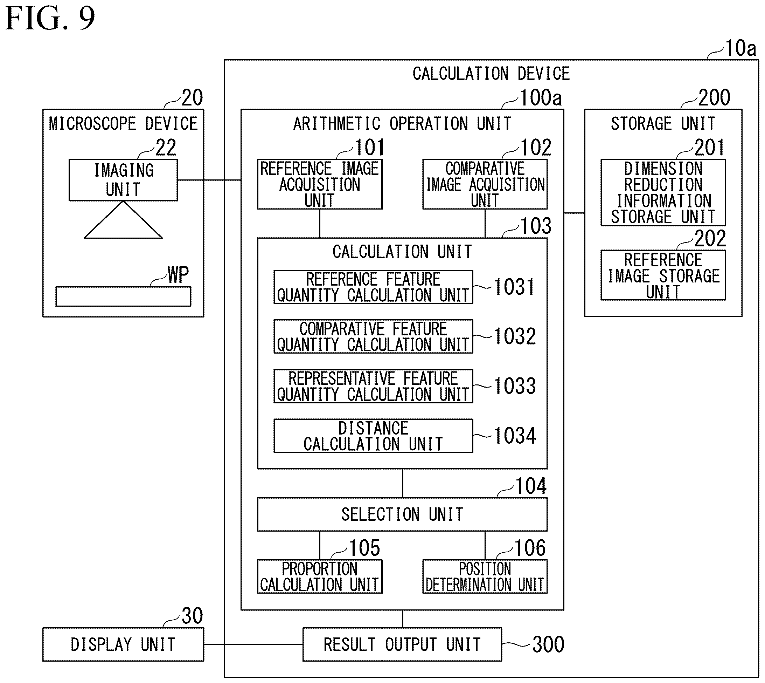

Fig. 9 is a block diagram showing an example of a functional configuration of units provided in a calculation device 10a according to the present embodiment. When the calculation device 10a (Fig. 9) according to the present embodiment is compared with the calculation device 10 (Fig. 2) according to the first embodiment, the presence or absence of a selection unit 104, a proportion calculation unit 105, and a position determination unit 106 is different. However, functions of the other components are the same as those of the first embodiment. A description of functions that are the same as those in the first embodiment will be omitted and parts different from those of the first embodiment will be mainly described in the second embodiment.

The selection unit 104 selects a comparative image in which a distance calculated by the distance calculation unit 1034 is larger than a predetermined value. The selection unit 104 supplies a selection result to the proportion calculation unit 105, the position determination unit 106, and a result output unit 300.

The proportion calculation unit 105 calculates a proportion of comparative images in which the distance calculated by the distance calculation unit 1034 is larger than the predetermined value from a plurality of comparative images on the basis of the selection result of the selection unit 104. The proportion calculation unit 105 supplies a calculation result to the result output unit 300.

The position determination unit 106 determines a position of a well W corresponding to the comparative image in which the distance calculated by the distance calculation unit 1034 is larger than the predetermined value on the basis of the selection result of the selection unit 104. The position determination unit 106 supplies a determination result to the result output unit 300.

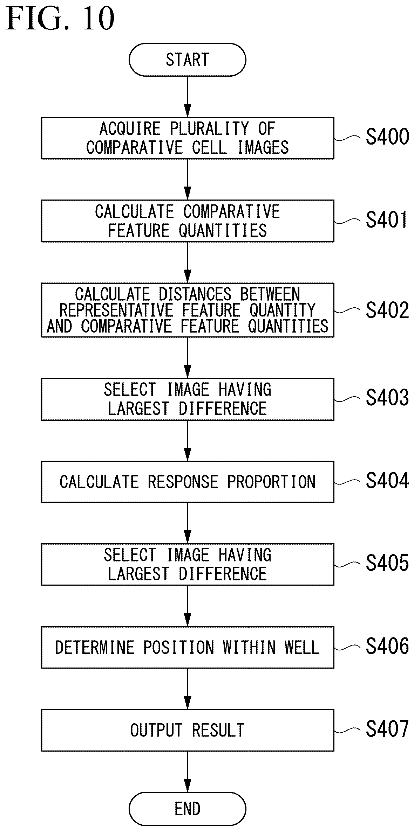

Fig. 10 is a flowchart showing an example of an arithmetic operation procedure of the arithmetic operation unit 100a for a plurality of comparative images according to the present embodiment. Also, because the processing of steps S400, S401, and S402 is similar to the processing of step S300, S301, and S302 in Fig. 7, respectively, a description thereof will be omitted.

The selection unit 104 selects a comparative image in which a distance calculated by the distance calculation unit 1034 is larger than a predetermined value (step S403). That is, the selection unit 104 selects a comparative image in which the difference calculated by the distance calculation unit 1034 is larger than a predetermined value. The selection unit 104 supplies information representing the selected comparative image to the proportion calculation unit 105.

The proportion calculation unit 105 calculates a proportion of comparative images in which a distance calculated by the distance calculation unit 1034 is larger than the predetermined value as a proportion of comparative images in which cells showing a response to an applied stimulus are imaged from the plurality of comparative images P21 to P29 on the basis of the information representing the comparative image supplied by the selection unit 104 (step S404). That is, the proportion calculation unit 105 calculates a proportion of comparative images in which the difference calculated by the calculation unit 103 is larger than the predetermined value from a plurality of comparative images using a selection result of the selection unit 104. The proportion calculation unit 105 supplies information representing the calculated proportion to the result output unit 300.

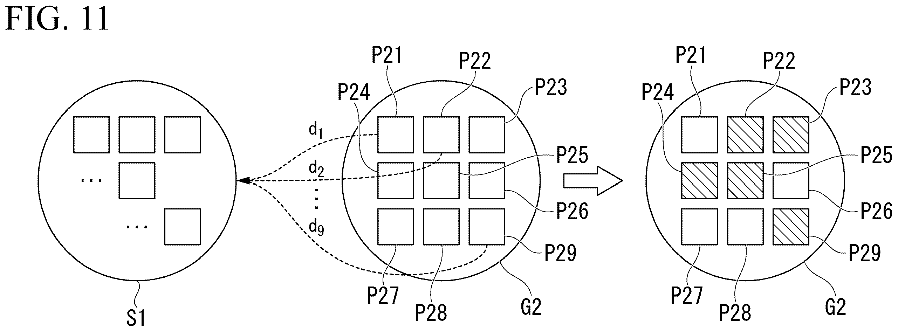

Here, a process of calculating a response proportion of a plurality of comparative images will be described with reference to Fig. 11.

Fig. 11 is a diagram showing an example of calculation of a response proportion of a plurality of comparative images according to the present embodiment. The comparative feature quantity calculation unit 1032 calculates a comparative feature quantity for each of the comparative images P21 to P29. The distance calculation unit 1034 calculates distances d₁ to d₉ from a reference representative feature quantity calculated from the reference image group S1 with respect to each of the comparative feature quantities calculated by the comparative feature quantity calculation unit 1032. The selection unit 104 determines a distance that is larger than or equal to a predetermined value among the distances d₁ to d₉ calculated by the distance calculation unit 1034. Here, for example, the predetermined value is 3.0. In an example shown in Fig. 11, d₂, d₃, d₄, d₅, and d₉ are larger than or equal to the predetermined value. The selection unit 104 selects the comparative image P22, the comparative image P23, the comparative image P24, the comparative image P25, and the comparative image P29, which correspond to d₂, d₃, d₄, d₅, and d₉, respectively, from among the comparative images P21 to P29. In the example shown in Fig. 11, the proportion calculation unit 105 calculates a proportion of the comparative images in which a distance is larger than the predetermined value as 5/9.

Returning to Fig. 10, a description of the arithmetic operation procedure of the arithmetic operation unit 100a for a plurality of comparative images will be continued.

The display unit 30 highlights and displays only comparative images having a distance larger than or equal to a predetermined threshold value among the wells including the plurality of comparative images. For example, in Fig. 11, the comparative images P22, P23, P24, P25, and P29 among the plurality of comparative images P21 to P29 are highlighted and displayed.

The selection unit 104 selects a comparative image having a maximum distance calculated by the distance calculation unit 1034 (step S405). The selection unit 104 outputs information representing a comparative image having a largest selected distance to the position determination unit 106 and the result output unit 300.

The position determination unit 106 determines a position of a well W corresponding to a comparative image having a maximum distance calculated by the distance calculation unit 1034 on the basis of information representing a comparative image having a maximum distance supplied by the selection unit 104 (step S406). That is, the position determination unit 106 determines a position corresponding to a comparative image in which the difference calculated by the calculation unit 103 is larger than a predetermined value using a selection result of the selection unit 104. In this regard, a plurality of comparative images correspond to a plurality of positions in a culture vessel in which cells are cultured, respectively. The position determination unit 106 supplies information representing the determined position of the well W to the result output unit 300.

The result output unit 300 outputs information representing an arithmetic operation result supplied by the arithmetic operation unit 100a to the display unit 30 (step S407). Here, the information representing the arithmetic operation result supplied by the arithmetic operation unit 100a is information representing the proportion supplied by the proportion calculation unit 105, information representing the selected comparative image supplied by the selection unit 104, and information representing the position of the well W supplied by the position determination unit 106.

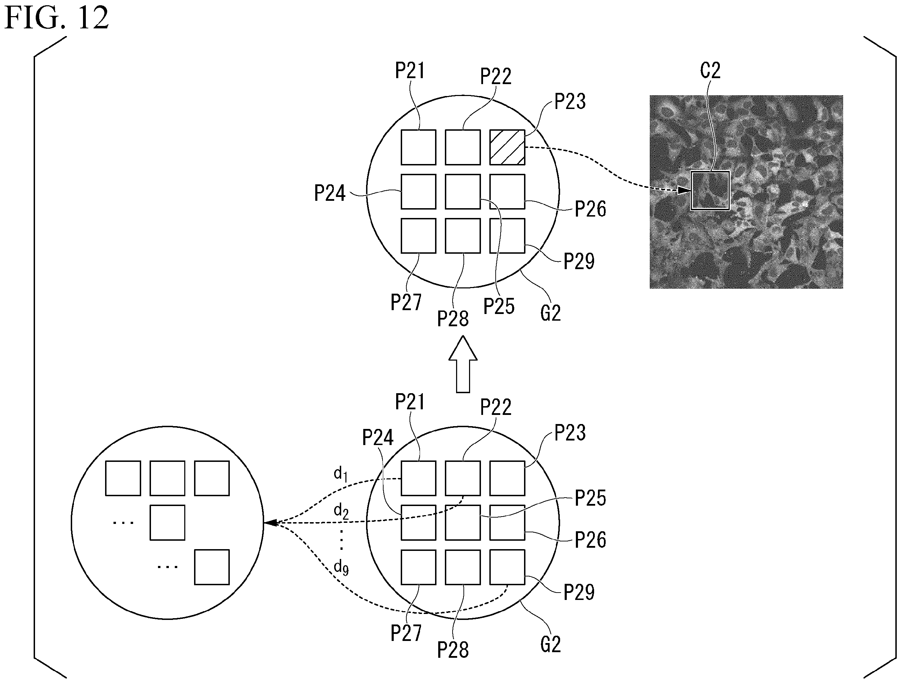

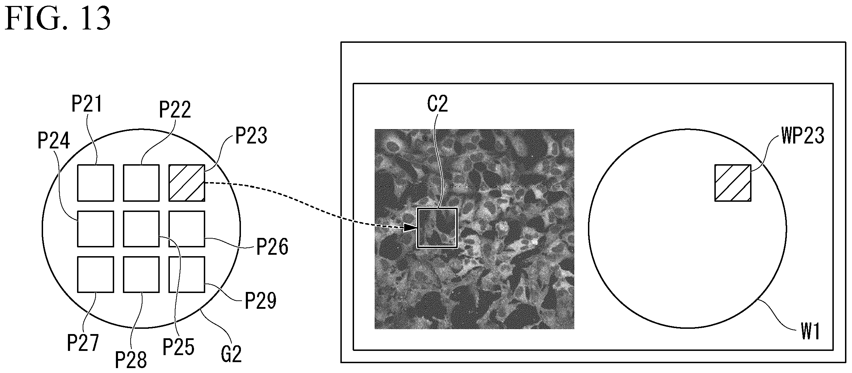

Here, a process of selecting a comparative image having the maximum distance and a process of determining a position within the well will be described with reference to Figs. 12 and 13.

Fig. 12 is a diagram showing an example of a process of selecting a comparative image according to the present embodiment. In the example shown in Fig. 12, d₃ is a largest distance among distances d₁ to d₉ calculated by the distance calculation unit 1034. The selection unit 104 selects a comparative image P23 as a comparative image having the maximum distance calculated by the distance calculation unit 1034. The display unit 30 displays the comparative image P23 as a cell image C2.

Fig. 13 is a diagram showing an example of a process of determining a position within a well according to the present embodiment. The position determination unit 106 determines a position WP23 within a well corresponding to a comparative image having a maximum distance from a reference image group S1 on the basis of information representing the comparative image P23 that is the comparative image having the maximum distance from the reference image group S1. The display unit 30 displays the position WP23 within the well.

As described above, the calculation device 10a of the present embodiment includes the selection unit 104, the proportion calculation unit 105, and the position determination unit 106.

The selection unit 104 selects a comparative image in which the difference calculated by the calculation unit 103 is larger than a predetermined value. According to this configuration, because it is possible to select a comparative image in which the difference from a plurality of reference images is larger than a predetermined value, it is possible to select a cell image of cells having a large response to an applied stimulus from among cell images.

The proportion calculation unit 105 calculates a proportion of comparative images in which the difference calculated by the calculation unit 103 is larger than a predetermined value from a plurality of comparative images using a selection result of an image selection unit (the selection unit 104). According to this configuration, because it is possible to calculate a proportion of comparative images in which the difference from a plurality of reference images is larger than the predetermined value, it is possible to calculate a proportion of cell images of cells having a large response to an applied stimulus from among cell images.

The plurality of comparative images correspond to a plurality of positions of the culture vessel in which cells are cultured, respectively, and the position determination unit 106 determines the position corresponding to the comparative image in which the difference calculated by the calculation unit is larger than a predetermined value using a selection result of the image selection unit. According to this configuration, because it is possible to determine a position within a well W for which a comparative image in which the difference from the plurality of reference images is larger than a predetermined value is captured, it is possible to determine a position where there is a cell having a large response to an applied stimulus among a plurality of cells within the well W.

### [Third embodiment]

Hereinafter, a third embodiment of the present invention will be described with reference to the drawings.

In the above-described second embodiment, a case in which the calculation device selects a comparative image in which a quantified difference between a reference image and a comparative image is larger than a predetermined value has been described. In the present embodiment, a case in which the calculation device calculates a change in time series, a change in a concentration of a compound added to cells, and a change in a type of compound with respect to the difference between a reference image group and a comparative image group will be described.

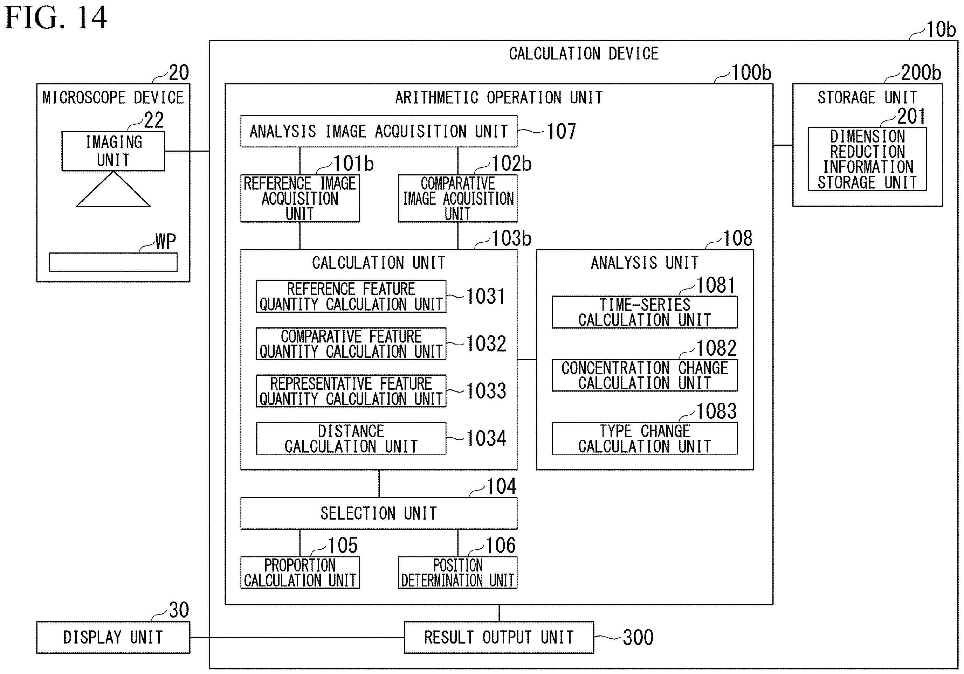

Fig. 14 is a block diagram showing an example of a functional configuration of units provided in a calculation device 10b according to the third embodiment of the present invention. When the calculation device 10b (Fig. 14) according to the present embodiment is compared with the calculation device 10a (Fig. 9) according to the second embodiment, there is a difference in that an analysis image acquisition unit 107, a reference image acquisition unit 101b, a comparative image acquisition unit 102b, a calculation unit 103b, and an analysis unit 108 in an arithmetic operation unit 100b are different and a storage unit 200b does not include the reference image storage unit 202. However, functions of the other components are the same as those of the second embodiment. A description of the functions which are the same as those of the second embodiment will be omitted and parts of the third embodiment different from those of the second embodiment will be mainly described.

The arithmetic operation unit 100b includes the reference image acquisition unit 101b, the comparative image acquisition unit 102b, the calculation unit 103b, the selection unit 104, a proportion calculation unit 105, a position determination unit 106, the analysis image acquisition unit 107, and the analysis unit 108.

The analysis image acquisition unit 107 acquires a group of analysis images captured by the imaging unit 22. This analysis image group includes a time-series analysis image group, a concentration change analysis image group, and a type change analysis image group.

The time-series analysis image group is a plurality of cell images obtained through time-lapse photographing of cells after cell stimulation. The time-series analysis image group includes an image group T0, an image group T1, ..., an image group Tn. The image group T0, the image group T1, ..., the image group Tn correspond to the time series of time-lapse photographing in that order. The image group T0, the image group T1, ..., the image group Tn may not be cell images of cells cultured in the same well. Also, each of the image group T0, the image group T1, ..., the image group Tn may be one cell image.

The analysis image acquisition unit 107 sets the image group TO as a time-series reference image group and supplies the time-series reference image group to the reference image acquisition unit 101b. The analysis image acquisition unit 107 sets the image group T1, the image group T2, ..., the image group Tn as a time-series comparative image group and supplies the time-series comparative image group to the comparative image acquisition unit 102b. That is, a comparative image included in the time series comparative image group is an image captured in time series.

The concentration change analysis image group is a plurality of cell images for each concentration of a compound added to cells. The concentration change analysis image group includes an image group X0, an image group X1, ..., an image group Xn. The image group X0, the image group X1, ..., the image group Xn correspond to the order in which the concentration of the compound added to the cells increases in that order. Also, each of the image group X0, the image group X1, ..., the image group Xn may be one cell image.

The analysis image acquisition unit 107 sets the image group X0 as a concentration-change reference image group and supplies the concentration-change reference image group to the reference image acquisition unit 101b. The analysis image acquisition unit 107 sets the image group X1, the image group X2, ..., the image group Xn as a concentration-change comparative image group and supplies the concentration-change comparative image group to the comparative image acquisition unit 102b. That is, the comparative image included in the concentration-change comparative image group is an image of comparative cells imaged for each concentration of the compound added to the comparative cells.

The type change analysis image group is a plurality of cell images for each type of compound added to cells. The type change analysis image group includes an image group Y0, an image group Y1, ..., an image group Yn. The image group Y0, the image group Y1, ..., the image group Yn correspond to the types of compounds added to the cells. Each of the image group Y0, the image group Y1, ..., the image group Yn may be one cell image.

The analysis image acquisition unit 107 sets the image group Y0 as the type-change reference image group and supplies the type-change reference image group to the reference image acquisition unit 101b. The analysis image acquisition unit 107 supplies the image group Y1, the image group Y2, ..., the image group Yn to the calculation unit 103b. That is, a comparative image included in a type-change comparative image group is an image of cells imaged for each type of compound added to comparative cells.

The reference image acquisition unit 101b acquires the time-series-change reference image group supplied by the analysis image acquisition unit 107 and supplies the time-series-change reference image group to the calculation unit 103b. The reference image acquisition unit 101b acquires the concentration-change reference image group supplied by the analysis image acquisition unit 107 and supplies the concentration-change reference image group to the calculation unit 103b. The reference image acquisition unit 101b acquires the type-change reference image group supplied by the analysis image acquisition unit 107 and supplies the type-change reference image group to the calculation unit 103b.

The comparative image acquisition unit 102b acquires the time-series-change comparative image group supplied by the analysis image acquisition unit 107 and supplies the time-series-change comparative image group to the calculation unit 103b. The comparative image acquisition unit 102b acquires the concentration-change comparative image group supplied by the analysis image acquisition unit 107 and supplies the concentration-change comparative image group to the calculation unit 103b. The comparative image acquisition unit 102b acquires the type-change reference image group supplied by the analysis image acquisition unit 107 and supplies the type-change reference image group to the calculation unit 103b.

The calculation unit 103b calculates distances between a reference representative feature quantity calculated on the basis of the image group TO which is the time-series reference image group supplied by the reference image acquisition unit 101b, and comparative representative feature quantities calculated on the basis of the image group T1, the image group T2, ..., the image group Tn which are the time-series analysis image groups supplied by the comparative image acquisition unit 102b as time-series distances. The calculation unit 103b supplies the calculated time-series distances to a time-series calculation unit 1081 of the analysis unit 108.

The calculation unit 103b calculates distances between a reference representative feature quantity calculated on the basis of the image group X0 which is the concentration-change reference image group supplied by the reference image acquisition unit 101b and comparative representative feature quantities calculated on the basis of the image group XI, the image group X2, ..., the image group Xn which are the concentration change analysis image groups supplied by the comparative image acquisition unit 102b as concentration change distances. The calculation unit 103b supplies the calculated concentration change distances to a concentration change calculation unit 1082 of the analysis unit 108.

The calculation unit 103b calculates distances between the reference representative feature quantity calculated on the basis of the image group Y0 which is the type-change reference image group supplied by the reference image acquisition unit 101b and the comparative representative feature quantities calculated on the basis of the image group Y1, the image group Y2, ..., the image group Yn which are the type change analysis image groups supplied by the comparative image acquisition unit 102b as type change distances. The calculation unit 103b supplies the calculated type change distances to a type change calculation unit 1083 of the analysis unit 108.

Also, if the comparative image group is one comparative image, the calculation unit 103 may not include the representative feature quantity calculation unit 1033.

The analysis unit 108 calculates a change in time series, a change in the concentration of a compound added to cells, and a change in a type of compound with respect to the difference between a reference image group and a comparative image group. The analysis unit 108 supplies the calculated change in time series, the calculated change in the concentration of the compound added to cells, and the calculated change in the type of compound to the result output unit 300. The analysis unit 108 includes the time-series calculation unit 1081, the concentration change calculation unit 1082, and the type change calculation unit 1083.

The time-series calculation unit 1081 calculates differences between a feature quantity calculated on the basis of the image group T0 and feature quantities calculated on the basis of the image group T1, the image group T2, ..., the image group Tn for each time in time series on the basis of the time-series distance supplied by the calculation unit 103b. That is, the time-series calculation unit 1081 calculates differences between feature quantities calculated on the basis of a plurality of reference images and feature quantities calculated on the basis of a plurality of comparative images for each time in time series using the differences supplied by the calculation unit 103b.

The concentration change calculation unit 1082 calculates differences between a feature quantity calculated on the basis of the image group X0 and feature quantities calculated on the basis of the image group XI, the image group X2, ..., the image group Xn for each concentration of a compound added to cells on the basis of the concentration change distances supplied by the calculation unit 103b. That is, the concentration change calculation unit 1082 calculates differences between feature quantities calculated on the basis of a plurality of reference images and feature quantities calculated on the basis of a plurality of comparative images for each concentration of the compound added to the comparative cells using the differences calculated by the calculation unit 103b.

The type change calculation unit 1083 calculates differences between a feature quantity calculated on the basis of the image group Y0 and feature quantities calculated on the basis of the image group Y1, the image group Y2, ..., the image group Yn for each type of compound added to cells on the basis of the type change distances supplied by the calculation unit 103b. That is, the type change calculation unit 1083 calculates differences between feature quantities calculated on the basis of a plurality of reference images and feature quantities calculated on the basis of a plurality of comparative images for each type of compound added to the comparative cells using the differences calculated by the calculation unit 103b.

Also, the proportion calculation unit 105 may calculate a proportion of comparative images in which a difference calculated by the calculation unit 103 is larger than a predetermined value from the comparative image group. For example, the proportion calculation unit 105 may calculate a proportion of comparative images in which a difference calculated by the calculation unit 103 is larger than a predetermined value from the time-series analysis image group for each time in time series. The proportion calculation unit 105 supplies the calculated proportion for each time in time series to the time-series calculation unit 1081. The proportion calculation unit 105 may calculate a proportion of comparative images in which a difference calculated by the calculation unit 103 is larger than a predetermined value from the concentration change analysis image group for each concentration of the compound added to the cells. The proportion calculation unit 105 supplies the calculated proportion for each concentration to the concentration change calculation unit 1082. The proportion calculation unit 105 may calculate a proportion of comparative images in which the difference calculated by the calculation unit 103 is larger than a predetermined value for each type of compound added to the cell from the type change analysis image group. The proportion calculation unit 105 supplies the calculated proportion for each type to the type change calculation unit 1083.

A process in which the calculation device 10b calculates the change in time series, the change in the concentration of the compound added to the cells, and the change in the type of compound with respect to the difference between the reference image group and the comparative image group will be described.

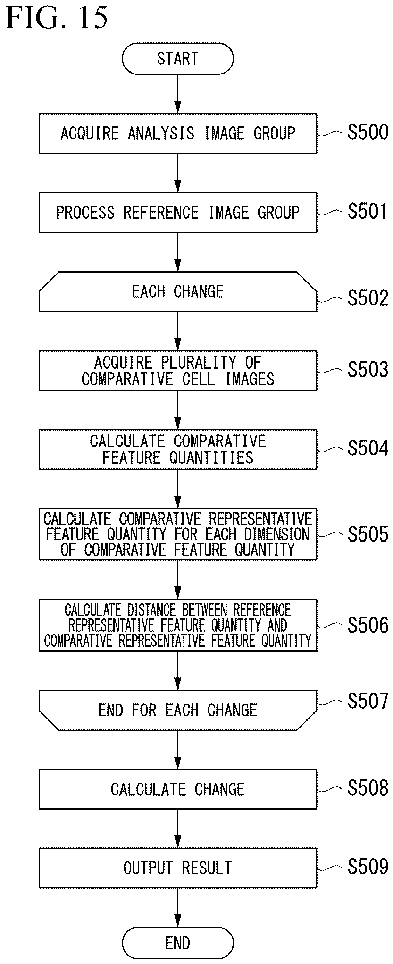

Fig. 15 is a flowchart showing an example of the arithmetic operation procedure of the arithmetic operation unit 100b of the present embodiment. Also, because the processing of step S503, step S504, step S505, and step S506 is similar to the processing of step S300, step S301, step S302, and step S303 in Fig. 7, respectively, a description thereof will be omitted.

The analysis image acquisition unit 107 acquires a type of analysis image group according to the change imaged by the imaging unit 22 (step S500). The analysis image acquisition unit 107 acquires one type of analysis image group from the time-series analysis image group, the concentration change analysis image group, and the type change analysis image group in accordance with a change analyzed by the calculation device 10b. A user of the calculation device 10b may designate one of the change in time series, the change in the concentration of the compound, and the change in the type of compound to be analyzed by the calculation device 10b.

When the time-series analysis image group has been acquired, the analysis image acquisition unit 107 sets the image group TO as the time-series reference image group and supplies the time-series reference image group to the reference image acquisition unit 101b. When the time-series analysis image group has been acquired, the analysis image acquisition unit 107 sets the image group T1, the image group T2,...., the image group Tn as the time-series comparative image group and supplies the time-series comparative image group to the comparative image acquisition unit 102b.

When the concentration change analysis image group has been acquired, the analysis image acquisition unit 107 sets the image group X0 as the concentration-change reference image group and supplies the concentration-change reference image group to the reference image acquisition unit 101b. When the concentration change analysis image group has been acquired, the analysis image acquisition unit 107 sets the image group XI, the image group X2, ..., the image group Xn as the concentration-change comparative image group and supplies the concentration-change comparative image group to the comparative image acquisition unit 102b.

When the type change analysis image group has been acquired, the analysis image acquisition unit 107 sets the image group Y0 as the type-change reference image group and supplies the type-change reference image group to the reference image acquisition unit 101b. When the type change analysis image group has been acquired, the analysis image acquisition unit 107 supplies the image group Y1, the image group Y2, ..., the image group Yn to the calculation unit 103b.

The reference image acquisition unit 101b and the calculation unit 103b perform processing on the reference image group (step S501). The processing to be performed on the reference image group here is similar to each of the processings of steps S100 to S102 of Fig. 3.

The calculation unit 103b starts a process for each change in accordance with the change to be analyzed (step S502). The calculation unit 103b iterates the processing of step S503, step S504, step S505, and step S506 for each change and calculates a distance between a reference representative feature quantity and a comparative representative feature quantity for each change.

When the change in time series is analyzed, the calculation unit 103 calculates a distance between a reference representative feature quantity calculated from the image group TO for each time in time series and a comparative representative feature quantity calculated from the time-series comparative image group corresponding to the time.

When the change in the concentration of the compound is analyzed, the calculation unit 103 calculates a distance between a reference representative feature quantity calculated from the image group X0 and a comparative representative feature quantity calculated from the concentration-change comparative image group corresponding to the concentration for each concentration of a compound.

When the change in the type of compound is analyzed, the calculation unit 103 calculates a distance between a reference representative feature quantity calculated from the image group Y0 and a comparative representative feature quantity calculated from the type-change comparative image group corresponding to the type for each type of compound.

The calculation unit 103b ends the process for each change (step S507). The calculation unit 103b supplies the calculated distance for each change to the analysis image acquisition unit 107.

The analysis unit 108 calculates a change in the difference between the reference image group and the comparative image group in accordance with the change analyzed by the calculation device 10b (step S508). Here, the change in the difference between the reference image group and the comparative image group is a set of the difference between the reference image group and the comparative image group and an index representing the change. The change is the distance between the representative reference feature quantity calculated from the reference image group and the comparative representative feature quantity calculated from the comparative image group. The index representing a change is an index representing a time in time series, a concentration of a compound, and a type of compound.

When the calculation device 10b analyzes the change in time series, the time-series calculation unit 1081 acquires a distance for each time in time series supplied by the calculation unit 103b. The time-series calculation unit 1081 supplies the acquired distance and a time in time series corresponding to the distance as a set to the result output unit 300.

When the calculation device 10b analyzes the change in the concentration of the compound, the concentration change calculation unit 1082 acquires a distance for each concentration of the compound supplied by the calculation unit 103b. The concentration change calculation unit 1082 supplies the acquired distance and a concentration corresponding to the distance as a set to the result output unit 300.

When the calculation device 10b analyzes the change in the type of compound, the type change calculation unit 1083 acquires a distance for each type of compound supplied by the calculation unit 103b. The type change calculation unit 1083 supplies the acquired distance and a type corresponding to the distance as a set to the result output unit 300.

The result output unit 300 outputs a result by causing the display unit 30 to display a set of the distance supplied by the analysis unit 108 and an index representing the change (step S509). The display unit 30 displays a graph in which the distance supplied by the analysis unit 108 is plotted with respect to the index representing the change.

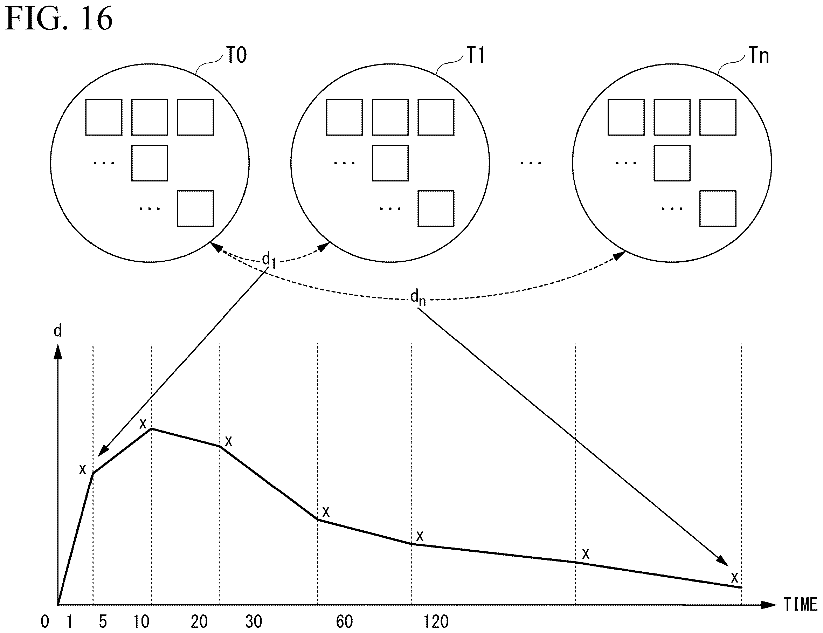

Here, an example of a change in the difference between the reference image group and the comparative image group calculated by the analysis unit 108 will be described with reference to Figs. 16 to 18.

Fig. 16 is a diagram showing an example of differences between a plurality of reference images and a plurality of comparative images for each time series according to the present embodiment. In Fig. 16, a graph in which differences between the image group TO which is the reference image group and the image group T1, the image group T2, ..., the image group Tn which are the comparative image groups corresponding to times in time series are plotted for the times is shown. The image group TO is a cell image in which cells are imaged immediately before the addition of the compound.

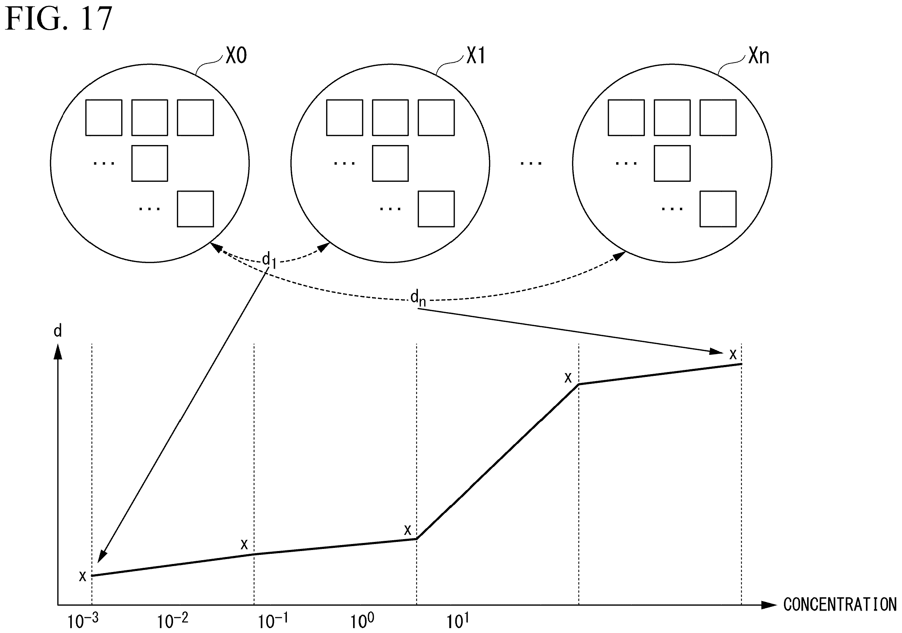

Fig. 17 is a diagram showing an example of differences between a plurality of reference images and a plurality of comparative images for each concentration of the compound according to the present embodiment. In Fig. 17, a graph in which differences between the image group X0 which is the reference image group and the image group XI, the image group X2, ..., the image group Xn which are the comparative image groups corresponding to concentrations of compounds are plotted for the concentrations is shown. The image group X0 is a cell image in which cells to which no compound has been added are imaged.

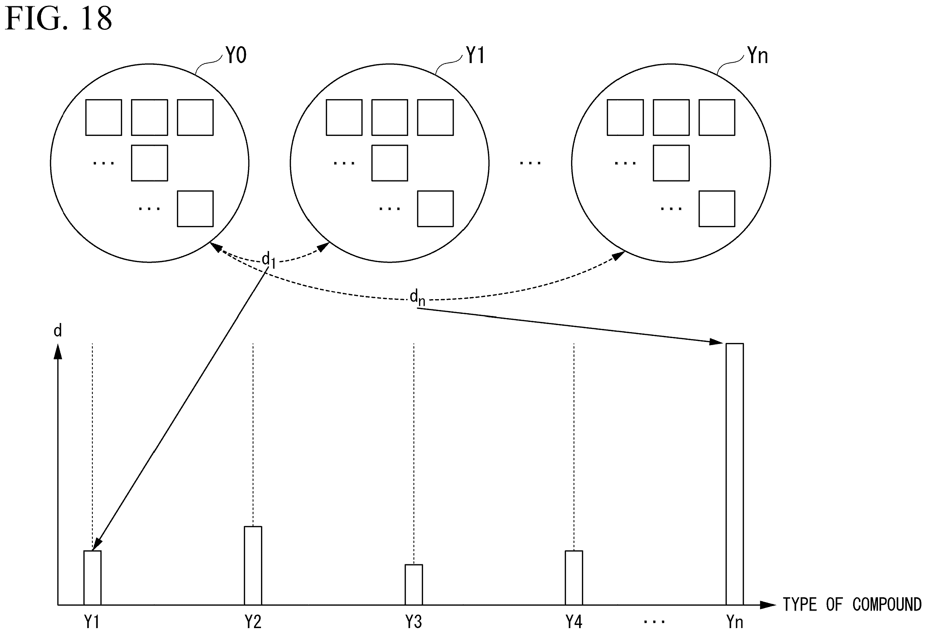

Fig. 18 is a diagram showing an example of differences between a plurality of reference images and a plurality of comparative images for each type of compound according to the present embodiment. In Fig. 18, a graph in which the difference between the image group Y0 which is the reference image group and the image group Y1, the image group Y2, ..., the image group Yn which are the comparative image groups corresponding to types of compounds are plotted for the types is shown. The image group Y0 is a cell image in which cells to which no compound has been added are imaged.

Also, although a case in which the calculation device 10b calculates a change in the difference between the reference image group and the comparative image group has been described in the present embodiment, the calculation device 10b may calculate a change in a response proportion. Here, the response proportion is a proportion of comparative images in which a distance from the reference image group is larger than a predetermined value among the plurality of comparative images included in the comparative image group. That is, the calculation device 10b may calculate a change in time series in the response proportion, a change in the response proportion with respect to a concentration of the compound, or a change in the response proportion with respect to a type of compound. The calculation device 10b may cause the display unit 30 to display the change in the calculated response proportion.

Also, the calculation device 10b may determine a position of a well W of the well plate WP corresponding to the comparative image in which a distance from the reference image group is larger than a predetermined value for each change. The calculation device 10b may cause the display unit 30 to display the determined position of the well W.

Also, although a case in which the calculation device 10b calculates differences between the image group Y0 which is the type-change reference image group and the image group Y1, the image group Y2, ..., the image group Yn which are the type-change comparative image groups when the change analyzed by the calculation device 10b is a change in the type of compound has been described in the present embodiment, the calculation device 10b may calculate a score of each of the image group Y1, the image group Y2, ..., the image group Yn. Here, the score means a value obtained by calculating an average value between distances after the distances between an image group whose score is to be calculated and image groups other than the image group whose score is to be calculated among the image group Y1, the image group Y2, ..., the image group Yn are obtained. The calculation device 10b may cause the display unit 30 to display a graph in which the calculated score is plotted for each type of compound.

Also, although a graph in which a distance between the reference image group and the comparative image group is plotted with respect to an index representing a change in accordance with a change analyzed by the calculation device 10b has been described with reference to Figs. 16 to 18 in the present embodiment, a result of combining two types of changes may be plotted on a two-dimensional plane. For example, the calculation device 10b may calculate a change in time series in the distance between the reference image group and the comparative image group for each concentration of a compound and may cause the display unit 30 to display a two-dimensional graph in which a calculation result is plotted with respect to the concentration of the compound and the time in time series. Also, for example, the calculation device 10b may calculate a change in time series in the distance between the reference image group and the comparative image group for each type of compound and cause the display unit 30 to display a two-dimensional graph in which a calculation result is plotted with respect to the type of compound and the time in time series. Also, for example, the calculation device 10b may calculate the distance between the reference image group and the comparative image group when the concentration of the compound is changed for each type of compound and cause the display unit 30 to display a two-dimensional graph in which a calculation result is plotted with respect to the type of compound and the concentration of the compound.

As described above, the calculation device 10b of the present embodiment includes the time-series calculation unit 1081, the concentration change calculation unit 1082, and the type change calculation unit 1083.

The time-series calculation unit 1081 calculates a difference for each time in the time series using a difference calculated by the calculation unit 103b. Here, the difference calculated by the calculation unit 103b is the difference between a feature quantity calculated on the basis of a plurality of reference images and a feature quantity calculated on the basis of a comparative image which is an image captured in time series. According to this configuration, it is possible to quantify a change in time series in a response of cells after the application of a stimulus because the calculation device 10b can quantify the difference between a cell image before the application of the stimulus and a cell image after the application of the stimulus in the time series after the stimulus is applied.

Also, the concentration change calculation unit 1082 calculates the difference for each concentration of a compound added to comparative cells using the difference calculated by the calculation unit 103b. Here, the difference calculated by the calculation unit 103b is the difference between a feature quantity calculated on the basis of a plurality of reference images and a feature quantity calculated on the basis of a comparative image which is an image of cells imaged for each concentration of the compound added to the comparative cells. According to this configuration, it is possible to quantify a response of cells to a concentration of an added compound because the calculation device 10b can quantify the difference between a cell image before the addition of the compound and a cell image after the addition of the compound with respect to a change in the concentration of the compound.

Also, the type change calculation unit 1083 calculates a difference for each type of compound added to comparative cells using the difference calculated by the calculation unit 103b. Here, the difference calculated by the calculation unit 103b is the difference between a feature quantity calculated on the basis of a plurality of reference images and a feature quantity calculated on the basis of a comparative image which is an image of cells imaged for each type of compound added to the comparative cells. According to this configuration, it is possible to quantify a response of cells to each type of added compound because the calculation device 10b can quantify the difference between a cell image before the addition of the compound and a cell image after the addition of the compound with respect to a change in the type of compound.

### [Fourth embodiment]

Hereinafter, a fourth embodiment of the present invention will be described with reference to the drawings.

In the above-described third embodiment, a case in which the calculation device calculates a change in time series, a change in a concentration of a compound added to cells, and a change in a type of compound with respect to the difference between a reference image group and a comparative image group has been described. In the present embodiment, a case in which the calculation device classifies cell images will be described.

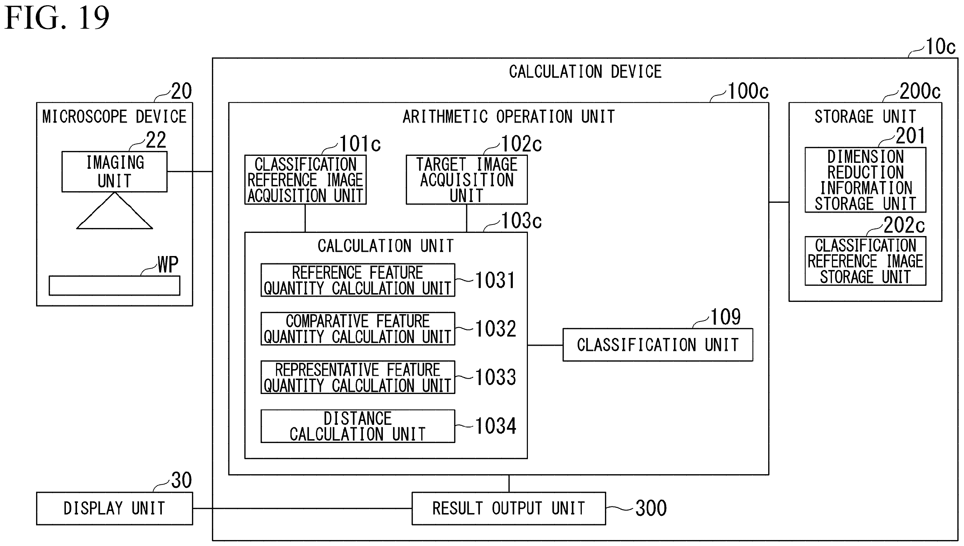

Fig. 19 is a block diagram showing an example of a functional configuration of units provided in a calculation device 10c according to the present embodiment. When the calculation device 10c (Fig. 19) according to the present embodiment is compared with the calculation device 10a (Fig. 9) according to the second embodiment, a classification reference image acquisition unit 101c, a target image acquisition unit 102c, a calculation unit 103c, and a classification unit 109 in an arithmetic operation unit 100c are different and a storage unit 200c is different. However, functions of the other components are the same as those of the second embodiment. A description of functions that are the same as those of the second embodiment will be omitted and parts of the third embodiment different from those of the second embodiment will be mainly described.

The classification reference image acquisition unit 101c acquires a classification reference image group stored in a classification reference image storage unit 202c of the storage unit 200c and supplies the acquired classification reference image group to the calculation unit 103c. Here, the classification reference image acquisition unit 101c acquires a classification reference image group corresponding to a class of a cell image classified by the calculation device 10c. That is, the classification reference image acquisition unit 101c acquires a plurality of types of reference images.

Here, the classification reference image group is a plurality of types of reference image groups for classifying cell images into classes. For example, a class into which a cell image is classified is a class classified according to each type of cell. For example, the type of cell may be a type of cell for each organ of an organism such as a heart cell or a brain cell. The type of cell may be a type of cell constituting a specific organ of an organism. Cells constituting a specific organ of an organism are, for example, astrocytes, glial cells, oligodendrocytes, and neurons constituting a nervous system. The type of cell may be epithelial cells or mesenchymal cells. The type of cell may be a cancer cell or a healthy cell. Also, a class into which a cell image is classified may be a class into which cells are classified according to each stage of differentiation. This class may be a class into which induced pluripotent stem cells (iPS) are classified for each stage of differentiation. Also, the class into which the cell image is classified may be a class into which cells are classified according to each division cycle.

Therefore, for example, when the calculation device 10c classifies cell images into an image of cancer cells and an image of healthy cells, the classification reference image acquisition unit 101c acquires a classification reference image group corresponding to cancer cells and a classification reference image group corresponding to healthy cells.

The target image acquisition unit 102c acquires one or more cell images captured by the imaging unit 22 as one or more target images and supplies the one or more target images that have been acquired to the calculation unit 103c.

The calculation unit 103c calculates distances between the classification reference image group supplied by the classification reference image acquisition unit 101c and the one or more target images supplied by the target image acquisition unit 102c. Here, the classification reference image group is a plurality of types of reference image groups and the plurality of types of reference image groups correspond to classes into which the calculation device 10c classifies cell images, respectively. The calculation unit 103c calculates the distance using the target image as a comparative image. That is, the calculation unit 103c calculates differences between a plurality of types of reference images and the comparative image. The calculation unit 103c supplies the distances corresponding to the calculated classes to the classification unit 109.

The classification unit 109 classifies the target image using a plurality of distances supplied by the calculation unit 103c. That is, the classification unit 109 classifies the comparative image using a plurality of differences calculated by the calculation unit.

The storage unit 200c includes a dimension reduction information storage unit 201 and a classification reference image storage unit 202c. The classification reference image storage unit 202c stores a plurality of types of classification reference image groups corresponding to the classes into which the calculation device 10c classifies cell images.

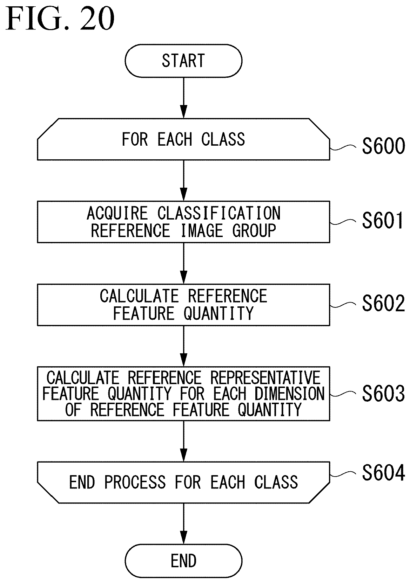

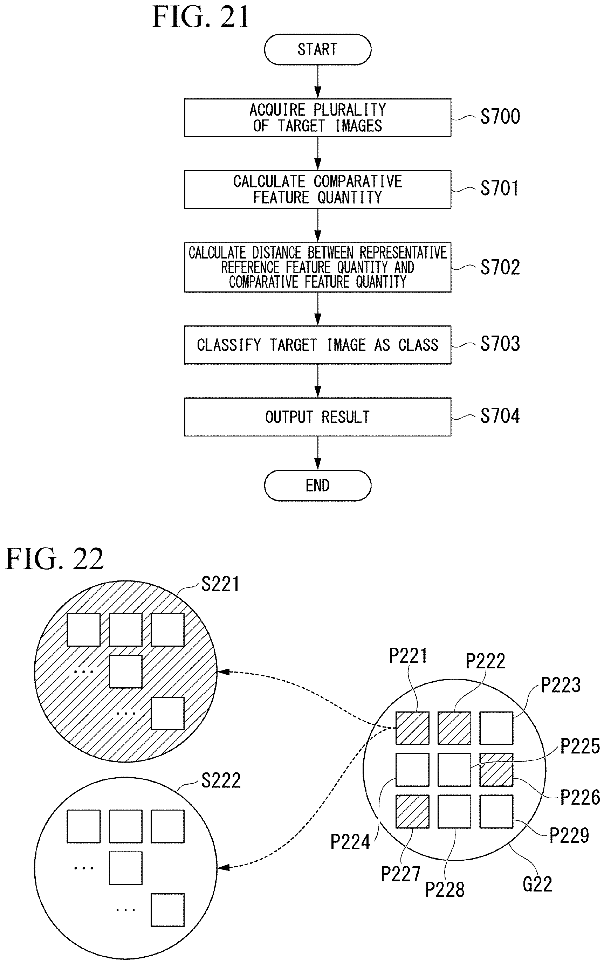

An arithmetic operation procedure of the arithmetic operation unit 100c will be described with reference to Figs. 20 and 21.

Fig. 20 is a flowchart showing an example of a procedure of calculating a reference feature quantity in the calculation unit according to the present embodiment.

The arithmetic operation unit 100c starts a process for each of classes into which the calculation device 10c classifies cell images (step S600).

The classification reference image acquisition unit 101c acquires a classification reference image group stored in the classification reference image storage unit 202c of the storage unit 200c (step S601). The classification reference image acquisition unit 101c supplies the acquired classification reference image group to the calculation unit 103c.

Because the processing of step S602 and step S603 is similar to the processing of step S101 and step S102 in Fig. 3, respectively, a description thereof will be omitted.

The arithmetic operation unit 100c ends the process for each class (step S604).

Fig. 21 is a flowchart showing an example of an arithmetic operation procedure of classifying target images into classes in the arithmetic operation unit according to the present embodiment.

The target image acquisition unit 102c acquires one or more cell images captured by the imaging unit 22 as one or more target images (step S700). The target image acquisition unit 102c supplies the one or more target images that have been acquired to the calculation unit 103c.

Because the processing of step S701 and step S702 is similar to the processing of step S201 and step S202 in Fig. 5, respectively, a description thereof will be omitted. In this regard, the calculation unit 103c calculates a distance from each of the plurality of classification reference image groups using the target image as the comparative image. The calculation unit 103c supplies distances corresponding to calculated classes to the classification unit 109.

Also, the calculation unit 103c may calculate a representative comparative feature quantity from a plurality of target images when the classification reference image acquisition unit 101c supplies the plurality of target images. The calculation unit 103c calculates a distance between the calculated representative comparative feature quantity and a representative reference feature quantity calculated from each of the plurality of types of reference image groups.

The classification unit 109 classifies the target image on the basis of a plurality of distances supplied by the calculation unit 103c (step S703). The classification unit 109 classifies the target image group into a class corresponding to the classification reference image group having a smallest distance from the classification reference image group. The classification unit 109 supplies a classification result to the result output unit 300.

Here, a process in which the classification unit 109 classifies target images will be described with reference to Fig. 22. Fig. 22 is a diagram showing an example of a process of classifying target images according to the present embodiment. In the example shown in Fig. 22, the calculation device 10c classifies a target image group G22 into two classes of cancer cells and healthy cells. A classification reference image group S221 is a reference image group in which cancer cells are imaged. A classification reference image group S222 is a reference image group in which healthy cells are imaged. The target image group G22 includes target images P221 to P229.

In a distance between the target image P221 and the classification reference image group S221 and a distance between the target image P221 and the classification reference image group S222, the distance between the target image P221 and the classification reference image group S221 is smaller. Therefore, the classification unit 109 classifies the target image P221 as a cancer cell class that is a class corresponding to the classification reference image group S221.

The calculation device 10c may classify each of the target images P221 to P229 included in the target image group G22 in order. In the example shown in Fig. 22, the target image P221, the target image P222, the target image P226, and the target image P227 are classified as images of cancer cells and the remaining target images are classified as images of healthy cells.

Returning to Fig. 21, a description of the arithmetic operation procedure of the arithmetic operation unit will be continued.

The result output unit 300 causes the display unit 30 to display a classification result supplied by the classification unit 109 (step S704).

As described above, the calculation device 10c of the present embodiment includes the classification reference image acquisition unit 101c, the calculation unit 103c, and the classification unit 109. The classification reference image acquisition unit 101c acquires a plurality of types of reference images. The calculation unit 103c calculates differences between the plurality of types of reference images and a comparative image. The classification unit 109 classifies the comparative image using a plurality of differences calculated by the calculation unit 103c. According to this configuration, it is possible to classify a cell image for each type of cell because the calculation device 10c can calculate differences between a plurality of types of reference images and a cell image.

### [Fifth embodiment]

Hereinafter, a fifth embodiment of the present invention will be described with reference to the drawings.

A case in which the calculation device calculates the difference between a reference image group and a comparative image group has been described in the above-described embodiment. A case in which a calculation device selects an abnormal image within a well or determines a cell culture state by calculating a distance between comparative image groups will be described in the present embodiment.

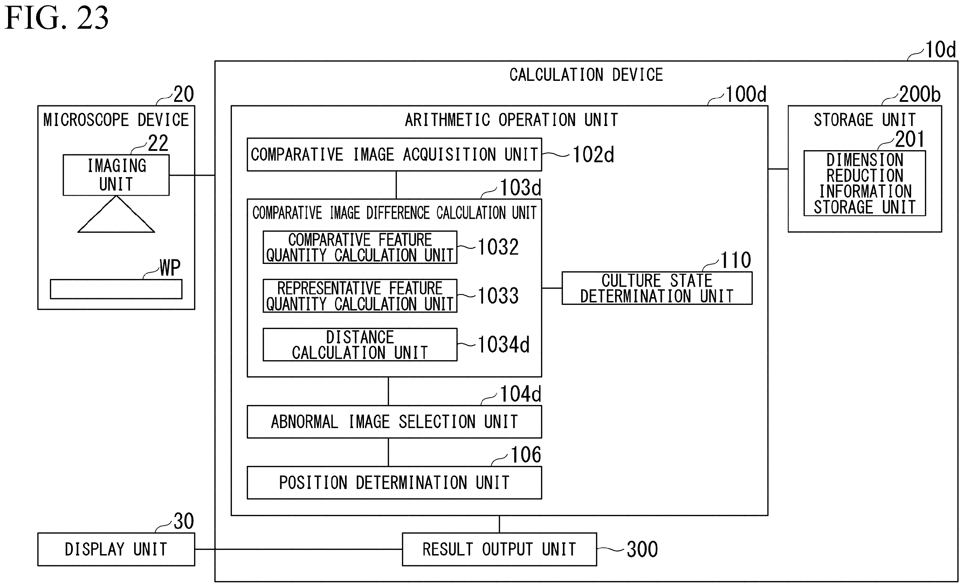

Fig. 23 is a block diagram showing an example of a functional configuration of units provided in a calculation device 10d according to the fifth embodiment of the present invention. When the calculation device 10d (Fig. 23) according to the present embodiment is compared with the calculation device 10a (Fig. 9) according to the second embodiment, a comparative image acquisition unit 102d, a comparative image difference calculation unit 103d, an abnormal image selection unit 104d, and a culture state determination unit 110 in an arithmetic operation unit 100d are different. Also, similar to the calculation device 10b (Fig. 14) according to the third embodiment, the storage unit 200b is different in that it is not necessary to provide a reference image storage unit. However, functions of the other components are the same as those of the second embodiment. A description of functions that are the same as those of the second embodiment will be omitted and parts of the fifth embodiment different from those of the second embodiment will be mainly described.

The comparative image acquisition unit 102d acquires a well comparative image group as a comparative image group with respect to a plurality of well plates imaged by the imaging unit 22 and supplies the acquired comparative image group to the comparative image difference calculation unit 103d. The well comparative image group is a plurality of cell images in which cells are imaged at each predetermined position of a well.

The comparative image difference calculation unit 103d calculates a distance between the comparative images included in the well comparative image group supplied by the comparative image acquisition unit 102d. That is, the comparative image difference calculation unit 103d calculates the difference between the comparative images. The comparative image difference calculation unit 103d includes a comparative feature quantity calculation unit 1032 and a distance calculation unit 1034d.

The distance calculation unit 1034d calculates an intra-image-group distance that is a distance from an image group including the other comparative images different from a comparative image with respect to each of the comparative images included in the well comparative image group supplied by the comparative image acquisition unit 102d. The distance calculation unit 1034d supplies the calculated intra-image-group distance to the abnormal image selection unit 104d.

The distance calculation unit 1034d divides a plurality of comparative images included in the well comparative image group into two comparative image groups and calculates an intra-well distance, which is a distance between the two comparative image groups. The distance calculation unit 1034d calculates an inter-well distance, which is a distance between the well comparative image groups for different wells. The distance calculation unit 1034d calculates an inter-plate distance, which is a distance between the well comparative image groups for wells of different well plates. The distance calculation unit 1034 supplies the calculated intra-well distance, the calculated inter-well distance, and the calculated inter-plate distance to the culture state determination unit 110.

The abnormal image selection unit 104d selects a comparative image in which an intra-image-group distance supplied by the comparative image difference calculation unit 103d is larger than a predetermined value as an abnormal image. That is, the abnormal image selection unit 104d selects a comparative image in which the difference between comparative images calculated by the comparative image difference calculation unit 103d is larger than a predetermined value among the comparative images as an abnormal image.

The abnormal image here is, for example, a cell image captured in the following cases. The abnormal image is, for example, a cell image in which dividing cells are imaged. The abnormal image is, for example, a cell image in which dead cells are imaged. The abnormal image is, for example, a cell image captured when a cell density within the well is extremely low. The abnormal image is, for example, a cell image captured in a state in which objects other than cells are mixed within the well as they are.

The culture state determination unit 110 determines a culture state of cells imaged in the comparative image on the basis of whether or not an intra-well distance, an inter-well distance, and an inter-plate distance supplied by the comparative image difference calculation unit 103d are within a predetermined range. That is, the culture state determination unit 110 determines the culture state of the comparative cells imaged in the comparative image on the basis of whether or not the difference between the comparative images is within a predetermined range.

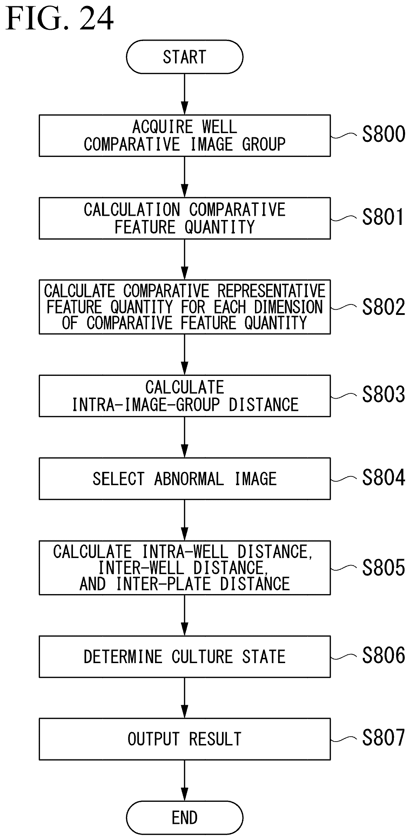

Now, a process in which the arithmetic operation unit 100d selects an abnormal image and a process of determining a cell culture state will be described with reference to Fig. 24.

Fig. 24 is a diagram showing an example of an arithmetic operation procedure of determining a culture state in the arithmetic operation unit 100d according to the present embodiment. Because the processing of step S801 and step S802 is similar to the processing of step S301 and step S302 in Fig. 7, respectively, a description thereof will be omitted.

The comparative image acquisition unit 102d acquires a well comparative image group for a plurality of well plates captured by the imaging unit 22 (step S800). The comparative image acquisition unit 102d supplies the acquired well comparative image group to the comparative image difference calculation unit 103d.

The distance calculation unit 1034d calculates an intra-image-group distance with respect to each of the comparative images included in the well comparative image group supplied by the comparative image acquisition unit 102d (step S803). The distance calculation unit 1034d supplies the calculated intra-image-group distance to the abnormal image selection unit 104d.

The abnormal image selection unit 104d selects a comparative image in which the intra-image-group distance supplied by the comparative image difference calculation unit 103d is larger than a predetermined value as an abnormal image (step S804). The abnormal image selection unit 104d supplies information representing the selected abnormal image to the result output unit 300. Also, the position determination unit 106 may determine the position of the abnormal image within the well on the basis of the information representing the abnormal image selected by the abnormal image selection unit 104d and supply the position to the result output unit 300.

The distance calculation unit 1034d calculates an intra-well distance, an inter-well distance, and an inter-plate distance (step S805). Here, the distance calculation unit 1034d divides a plurality of comparative images included in the well comparative image group into two comparative image groups and calculates the intra-well distance. Here, when the distance calculation unit 1034d divides the plurality of comparative images included in the well comparative image group into two comparative image groups, the comparative images are divided, for example, so that the number of comparative images included in the two comparative image groups is equalized. Here, for example, if the number of comparative images included in the well comparative image group is an odd number, the distance calculation unit 1034d divides comparative images so that the difference in the number of comparative images included in the two comparative image groups is one. Also, for example, the distance calculation unit 1034d classifies images at adjacent positions within the well as the same comparative image group. The distance calculation unit 1034d may classify images at positions, which are not adjacent to each other within the well if possible, into the same comparative image group.

The distance calculation unit 1034d calculates an inter-well distance between the well comparative image groups for all wells. For example, the distance calculation unit 1034d may select one or all positions from each well and calculate an inter-well distance between the well comparative image groups with respect to the selected position.

The distance calculation unit 1034d calculates an inter-plate distance between the well comparative image groups for all well plates. For example, the distance calculation unit 1034d may select one well from each well plate, further select one or all positions from the selected wells, and calculate an inter-plate distance between the well comparative image groups with respect to the selected position. For example, the distance calculation unit 1034d may select all wells from each well plate, select one or all positions from each selected well, and calculate an inter-plate distance between the well comparative image groups with respect to each selected position.

The distance calculation unit 1034d supplies the culture state determination unit 110 with the intra-well distance, the inter-well distance, and the inter-plate distance that have been calculated.

The culture state determination unit 110 determines a culture state of cells imaged in the comparative image on the basis of whether or not the intra-well distance, the inter-well distance, and the inter-plate distance supplied by the distance calculation unit 1034d are within a predetermined range (step S806). The culture state determination unit 110 supplies a determination result to the result output unit 300.

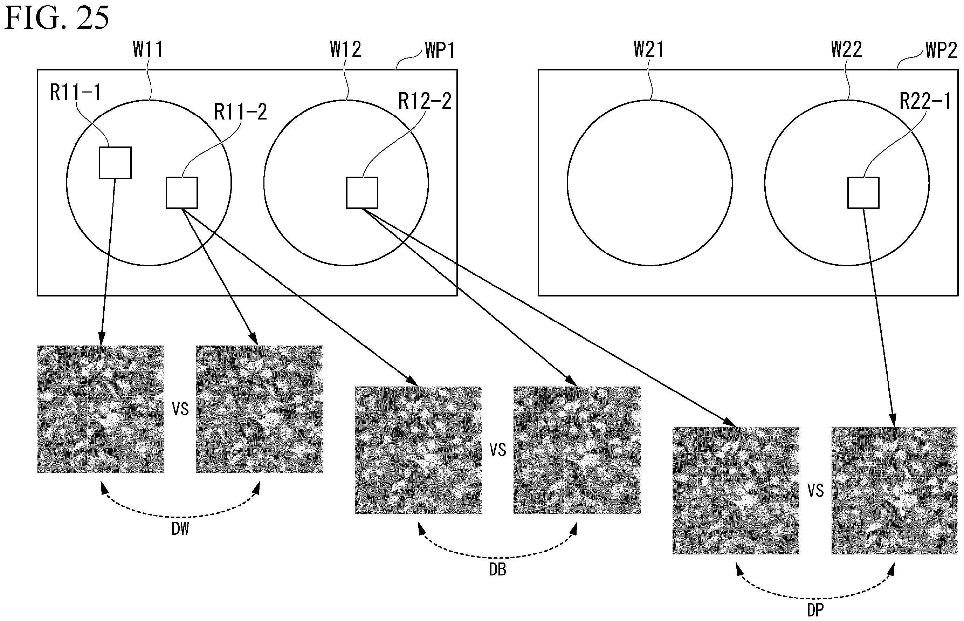

Now, a culture state determination process of the culture state determination unit 110 will be described with reference to Fig. 25.

Fig. 25 is a diagram showing an example of the culture state determination process according to the present embodiment. For example, the culture state determination unit 110 determines whether or not all of an intra-well distance DW, an inter-well distance DB, and an inter-plate distance DP are smaller than or equal to a predetermined threshold value. The culture state determination unit 110 determines that the culture state is appropriate when all of the intra-well distance DW, the inter-well distance DB, and the inter-plate distance DP are determined to be smaller than or equal to the predetermined threshold value.

For example, the culture state determination unit 110 may compare magnitudes of the intra-well distance DW, the inter-well distance DB, and the inter-plate distance DP and determine whether or not the intra-well distance DW, the inter-well distance DB, and the inter-plate distance DP increase in that order. The culture state determination unit 110 determines that the culture state is appropriate when the intra-well distance DW, the inter-well distance DB, and the inter-plate distance DP are determined to increase in that order.

For example, the culture state determination unit 110 may determine whether or not the inter-well distance DB for the wells within the well plate WP1 is smaller than or equal to a reference value using the inter-well distance DB for wells W11 and W12, which are a set of wells of the well plate WP1, as the reference value. The culture state determination unit 110 determines that the culture state is appropriate when the inter-well distance DB for the wells within the well plate WP1 is determined to be smaller than or equal to the reference value,.

For example, the culture state determination unit 110 may calculate an average of the inter-well distance DB between a certain well W11 of the well plate WP1 and the other well of the well plate WP1 with respect to the other wells of the well plate WP1 as a score of the well W11 and determine the culture state on the basis of the score. The culture state determination unit 110 calculates scores for all the wells within the well plate WP1 and calculates an average value of the scores for all the wells of the well plate WP1. The culture state determination unit 110 determines whether or not differences between the scores of all the wells within the well plate WP1 and the average value of the scores are within a predetermined threshold value. The culture state determination unit 110 determines that the culture state is appropriate when the differences between the scores of all the wells in the well plate WP1 and the average value of the scores are determined to be within the predetermined threshold value.

Returning to Fig. 24, a description of a process of the arithmetic operation unit 100d will be continued.

The result output unit 300 causes the display unit 30 to display a result supplied by the arithmetic operation unit 100d (step S807). The result output unit 300 causes the display unit 30 to display an abnormal image on the basis of information representing the abnormal image supplied by the abnormal image selection unit 104d. The result output unit 300 may cause the display unit 30 to display a position within the well of the abnormal image supplied by the position determination unit 106. The result output unit 300 causes the display unit 30 to display a determination result of a culture state supplied by the culture state determination unit 110.

Also, although a case in which the abnormal image selection unit 104d selects an abnormal image on the basis of an intra-image-group distance calculated for each comparative image included in the comparative images included in the well comparative image group has been described in the present embodiment, the abnormal image selection unit 104d may select the abnormal well on the basis of an inter-well distance. When it is determined whether a certain well AW within the well plate is to be selected as an abnormal image, for example, the representative feature quantity calculation unit 1033 calculates representative comparative feature quantities of the well comparative image groups for all other wells within the well plate other than a well AW. The representative feature quantity calculation unit 1033 calculates a representative value of a distribution of a plurality of representative comparative feature quantities that have been calculated as a plate representative feature quantity. The distance calculation unit 1034d calculates a distance between the comparative representative feature quantity of the well comparative image group for the well AW and the plate representative feature quantity. The abnormal image selection unit 104d determines that the well AW is an abnormal well when the distance between the comparative representative feature quantity of the well comparative image group for the well AW and the plate representative feature quantity is larger than or equal to a predetermined value.

Also, the distance calculation unit 1034d may calculate a sum of differences between distances of the comparative representative feature quantity of the well comparative image group for the well AW and the comparative representative feature quantities of the well comparative image groups for all other wells within the well plate other than the well AW with respect to all the other wells. The abnormal image selection unit 104d may determine the well AW as an abnormal well when the sum is larger than or equal to a predetermined value.

Also, when an abnormal image is selected, the comparative image acquisition unit 102 may acquire a plurality of comparative images instead of the well comparative image. The abnormal image selection unit 104d may select the abnormal image from among the plurality of comparative images acquired by the comparative image acquisition unit 102.

Also, although a case in which the well comparative image group is used to determine the culture state of cells has been described in the present embodiment, the determination of the culture state of the cells may be made using one cell image at a certain position within the well as a comparative image instead of the well comparative image group.

Also, although a case in which a distance between the representative feature quantities of the well comparative image groups is used for the determination of the culture state of the cells has been described in the present embodiment, a response proportion in the well comparative image group may be used to determine the culture state of the cells instead of the distance between the representative feature quantities of the well comparative image groups. When the response proportion in the well comparative image group is used to determine the culture state of the cells, the calculation device 10d may include the proportion calculation unit 105. For example, the response proportions in the well comparative image group are compared with respect to a plurality of positions within the well and it may be calculated whether the response proportion at a certain position is within a predetermined range as compared with an average value of the response proportions at other positions within the well. The calculation device 10d may determine that the culture state is appropriate when the response proportion is within the predetermined range with respect to each position within the well as compared with the average value of the response proportions at other positions within the well.

Alternatively, the response proportions in the well comparative image group may be compared with respect to a plurality of wells within the well plate and it may be calculated whether the response proportion of a certain well is within a predetermined range as compared with the average value of the response proportions of other wells within the well plate. When the response proportion is within the predetermined range as compared with the average value of the response proportions of the other wells within the well plate with respect to each well within the well plate, the culture state may be determined to be appropriate.

Alternatively, the response proportions in the well comparative image group are compared with respect to a plurality of well plates within the whole well plate and it may be calculated whether the response proportion of a certain well plate is within a predetermined range as compared with the average value of the response proportions of other well plates in the whole well plate. When the response proportion of a certain well plate is within the predetermined range as compared with the average value of the response proportions of other well plates within the whole well plate, the culture state may be determined to be appropriate.

As described above, the calculation device 10d of the present embodiment includes the comparative image difference calculation unit 103d and the abnormal image selection unit 104d. The comparative image difference calculation unit 103d calculates the difference between comparative images. The abnormal image selection unit 104d selects a comparative image in which the difference between the comparative images calculated by the comparative image difference calculation unit 103d is larger than a predetermined value among the comparative images as an abnormal image. According to this configuration, it is possible to select the abnormal image from among cell images corresponding to positions within the well because the calculation device 10d can compare a value obtained by quantifying the difference between the comparative images with a predetermined value.

Also, the calculation device 10d of the present embodiment includes a culture state determination unit 110. The culture state determination unit 110 determines the culture state of comparative cells imaged in the comparative image on the basis of whether or not the difference between comparative images is within a predetermined range. According to this configuration, it is possible to determine whether or not the culture state is appropriate for each well, each well plate, or all of a plurality of well plates because the calculation device 10d can determine whether or not a value obtained by quantifying the difference between comparative images is within a predetermined range.

### [Modified example of fifth embodiment]

Although a case in which the calculation device 10d calculates the difference between cell images of cells cultured in a well has been described in the above-described fifth embodiment, a case in which the calculation device 10d calculates the difference in a spheroid as an example of a cell cluster (colony) aggregated three-dimensionally will be described as a modified example. Hereinafter, parts different from those of the above-described fifth embodiment will be mainly described. An example of a three-dimensionally aggregated cell cluster other than a spheroid may be an organoid cultured in the form of a tissue.

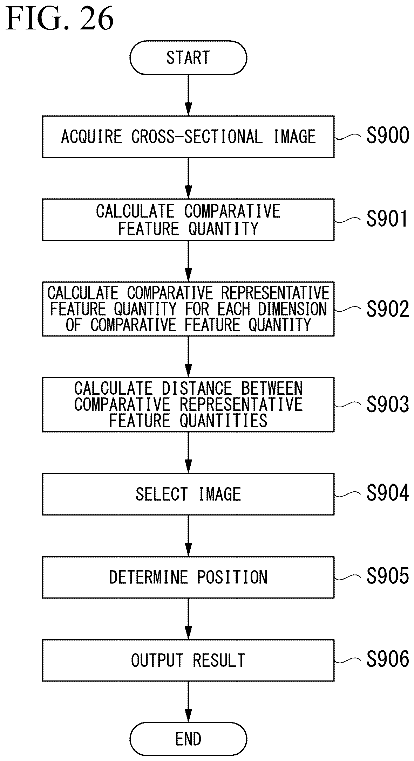

Fig. 26 is a diagram showing a modified example of an arithmetic operation procedure of the arithmetic operation unit 100d according to the present embodiment. Also, because the processing of steps S901 and S902 is similar to the processing of steps S801 and S802 in Fig. 24, respectively, a description thereof will be omitted.

The comparative image acquisition unit 102d acquires cross-sectional images PZ0 to PZn in which cross sections of the spheroid are imaged (step S900).

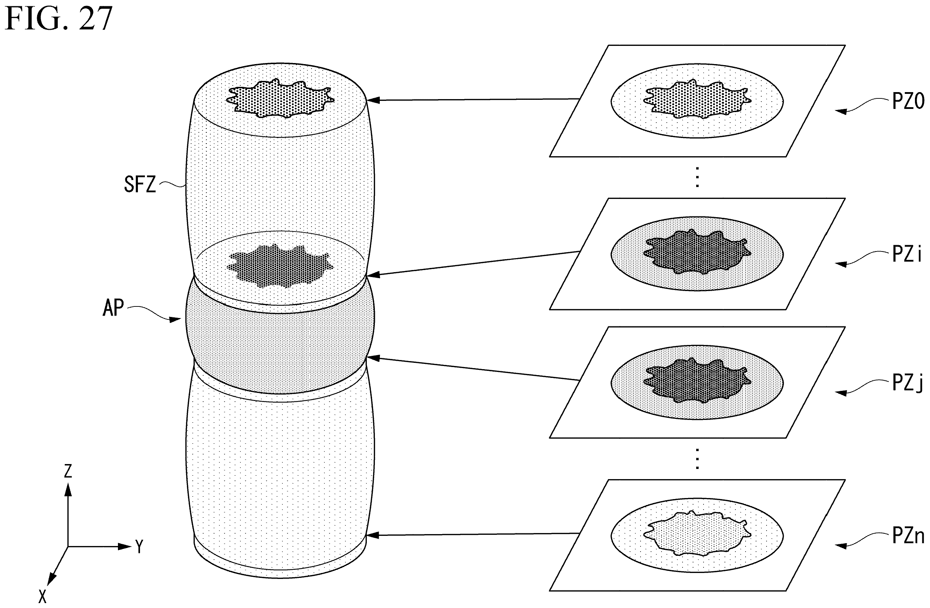

Here, the cross-sectional images of the spheroid will be described with reference to Fig. 27.

Fig. 27 is a diagram showing an example of a cross-sectional image of a spheroid according to the present embodiment. The spheroid is a cell cluster (colony) that is three-dimensionally aggregated. In the spheroid, it is possible to reproduce an environment closer to that in a living body as compared with two-dimensional culture. In Fig. 27, a direction-uniform spheroid image SFZ that is a three-dimensional image in which the spheroid expected to be uniform in the Z-axis direction is imaged is shown. However, in the direction-uniform spheroid image SFZ, a region AP has different properties from other regions. Cross-sectional images PZ0 to PZn are two-dimensional images for which cross sections corresponding to positions of the Z axis of the direction-uniform spheroid image SFZ are extracted. A cross-sectional image PZi and a cross-sectional image PZj are two-dimensional images for which cross sections corresponding to an upper surface and a lower surface in the Z-axis direction of the region R of the direction-uniform spheroid image SFZ are extracted. The number of cross-sectional images PZ0 to PZn is, for example, 1000.

Returning to Fig. 26, a description of an arithmetic operation procedure of the arithmetic operation unit 100d will be continued.

The comparative image acquisition unit 102d classifies each of the acquired cross-sectional images PZ0 to PZn as a predetermined region and supplies the comparative feature quantity calculation unit 1032 with the cross-sectional images PZ0 to PZn as a comparative image group including a plurality of regions.

The distance calculation unit 1034d calculates a distance between comparative representative feature quantities calculated by the representative feature quantity calculation unit 1033d (step S903). Here, the distance calculation unit 1034d calculates distances between the comparative representative feature quantities with respect to all combinations of the comparative representative feature quantities calculated from the cross-sectional images PZ0 to PZn. The distance calculation unit 1034d supplies a plurality of distances that have been calculated to the abnormal image selection unit 104d.

The abnormal image selection unit 104d selects a cross-sectional image having a distance that is larger than or equal to a predetermined value from the cross-sectional images PZ0 to PZn on the basis of the plurality of distances supplied by the distance calculation unit 1034d (step S904). In the example shown in Fig. 27, the abnormal image selection unit 104d selects a cross-sectional image PZi and a cross-sectional image PZj that are different from other regions of the direction-uniform spheroid image SFZ. The abnormal image selection unit 104d supplies the position determination unit 106 with information representing the selected cross-sectional images.

The position determination unit 106 determines a position within an image of the three-dimensional spheroid of the cross-sectional image on the basis of the information representing the cross-sectional images supplied by the abnormal image selection unit 104d (step S905). The position determination unit 106 supplies information representing the determined position to the result output unit 300.

The result output unit 300 causes the display unit 30 to display a position of a region different from other regions within the image of the three-dimensional spheroid on the basis of the information representing the position supplied by the position determination unit 106 (step S906).

### [Sixth embodiment]

Hereinafter, a sixth embodiment of the present invention will be described with reference to the drawings.

Although a case in which the calculation device quantifies the difference between cell images corresponding to positions within a well has been described in the above-described embodiment, a case in which a calculation device quantifies the difference between three-dimensional images in which a spheroid is imaged will be described in the present embodiment.

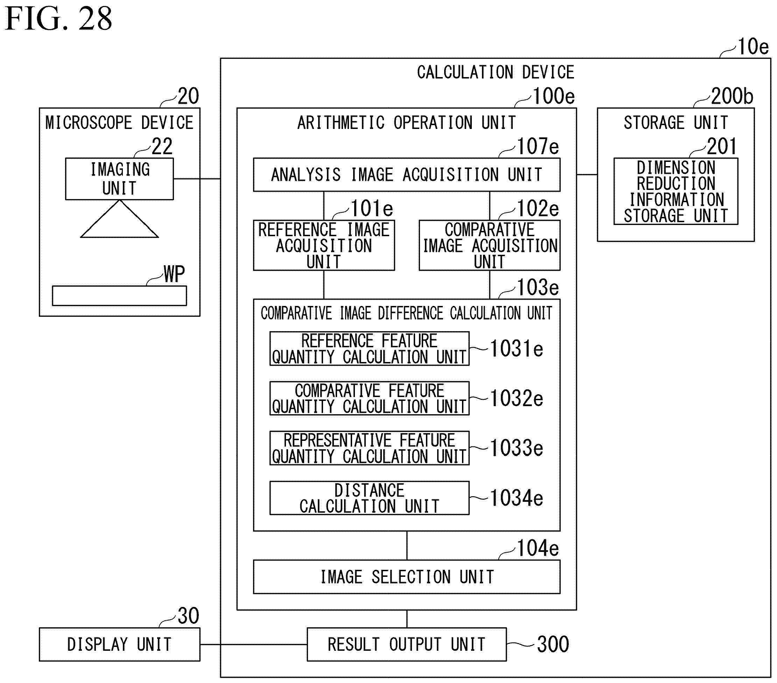

Fig. 28 is a block diagram showing an example of a functional configuration of units provided in a calculation device 10e according to the sixth embodiment of the present invention. When the calculation device 10e (Fig. 28) according to the present embodiment is compared with the calculation devices according to the first to fifth embodiments, there is a difference in that a cell image for which the arithmetic operation unit 100e calculates a difference is a three-dimensional spheroid image. A function of the calculation device 10e (Fig. 28) according to the present embodiment is similar to that of the fifth embodiment, except that the difference between the three-dimensional spheroid images is calculated. Descriptions of functions that are the same as those of the fifth embodiment will be omitted and parts of the sixth embodiment different from those of the fifth embodiment will be mainly described.

An analysis image acquisition unit 107e acquires an analysis image group captured by an imaging unit 22. This analysis image group is a spheroid image group which is a plurality of spheroid images. The spheroid image is a set of a plurality of voxels that can be obtained by extracting a predetermined number of voxels having a predetermined size from each of three-dimensional images in which spheroids produced in a similar technique are imaged. Here, the predetermined size is, for example, 100×100×100 pixels, and the predetermined number is, for example, 5×5×5. Therefore, the spheroid image group is a set in which a plurality of sets, each of which is a plurality of voxels extracted from the three-dimensional image of one spheroid, are further collected.

The analysis image acquisition unit 107e includes a spheroid image SF0, a spheroid image SF1, a spheroid image SF2, and a spheroid image SF3.

The analysis image acquisition unit 107e sets the spheroid image SF0 as a reference image group and supplies the reference image group to a reference image acquisition unit 101e. The analysis image acquisition unit 107e supplies a comparative image acquisition unit 102e with the spheroid image SF1, the spheroid image SF2, and the spheroid image SF3 as a comparative image group.

The reference image acquisition unit 101e acquires the reference image group supplied by the analysis image acquisition unit 107e and supplies the reference image group to a comparative image difference calculation unit 103e.

The comparative image acquisition unit 102e acquires the comparative image group supplied by the analysis image acquisition unit 107e and supplies the comparative image group to the comparative image difference calculation unit 103e.

The comparative image difference calculation unit 103e calculates the difference between a reference image group that is a plurality of three-dimensional images and a comparative image group that is a plurality of three-dimensional images. The comparative image difference calculation unit 103e includes a reference feature quantity calculation unit 1031e, a comparative feature quantity calculation unit 1032e, a representative feature quantity calculation unit 1033e, and a distance calculation unit 1034e.

The reference feature quantity calculation unit 1031e calculates feature quantities of voxels included in the spheroid image SF0, which is the reference image group supplied by the reference image acquisition unit 101e, as a plurality of reference feature quantities. Here, the voxel feature quantity is a tensor that can be obtained by classifying a voxel as a cross-sectional view that is a two-dimensional image at predetermined intervals along a certain axis and combining feature quantities calculated with respect to cross-sectional views as a set. Hereinafter, the voxel feature quantity may be referred to as a feature quantity tensor. The reference feature quantity calculation unit 1031e supplies the representative feature quantity calculation unit 1033e with a plurality of feature quantity tensors calculated for each voxel of the spheroid image SF0.

The comparative feature quantity calculation unit 1032e calculates feature quantity tensors of the spheroid images included in the spheroid image SF1, the spheroid image SF2, and the spheroid image SF3, which are the comparative image group supplied by the reference image acquisition unit 101e, as a plurality of comparative feature quantities. The comparative feature quantity calculation unit 1032e supplies the representative feature quantity calculation unit 1033e with a plurality of comparative feature quantities calculated with respect to each voxel of the spheroid image SF1, the spheroid image SF2, and the spheroid image SF3.

The representative feature quantity calculation unit 1033e calculates a representative feature quantity tensor from a plurality of feature quantity tensors of the spheroid image SF0 supplied by the reference feature quantity calculation unit 1031e, and sets the representative feature quantity tensor as a reference representative feature quantity tensor. Here, the representative feature quantity tensor is a tensor including a representative value of a distribution for each component of the plurality of feature quantity tensors. Here, the representative value is, for example, a median value or an average value. The representative feature quantity calculation unit 1033e supplies the calculated reference representative feature quantity tensor to the distance calculation unit 1034e.

The representative feature quantity calculation unit 1033e calculates a representative feature quantity tensor from a plurality of feature quantity tensors for each of the spheroid image SF1, the spheroid image SF2, and the spheroid image SF3 supplied by the comparative feature quantity calculation unit 1032e and sets the representative feature quantity tensor as a comparative representative feature quantity tensor. The representative feature quantity calculation unit 1033e supplies the comparative representative feature quantity tensor that has been calculated to the distance calculation unit 1034e.

The distance calculation unit 1034e calculates distances between the reference representative feature quantity tensor and a plurality of comparative representative feature quantity tensors supplied by the representative feature quantity calculation unit 1033e and supplies the calculated distances to an image selection unit 104e.

The image selection unit 104e selects a spheroid image from the comparative image group on the basis of a plurality of distances supplied by the distance calculation unit 1034e. The image selection unit 104e supplies the selected spheroid image to the result output unit 300.

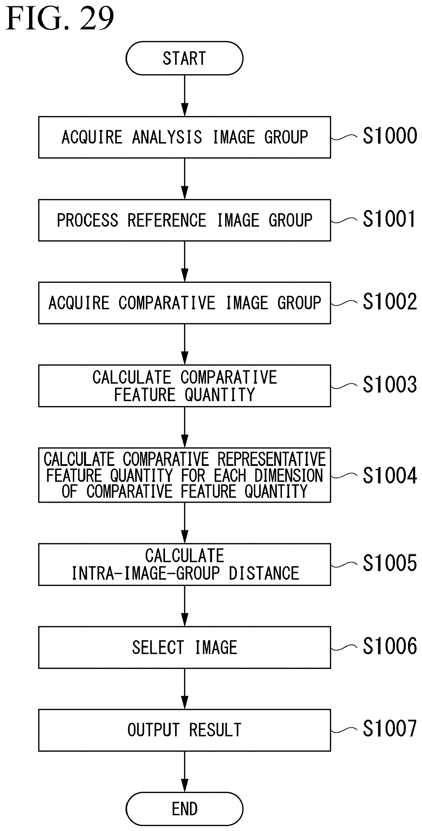

Now, a process in which the arithmetic operation unit 100e selects a spheroid image having a smallest difference from spheroid images which are the reference image group from the comparative image group will be described with reference to Fig. 29.

Fig. 29 is a diagram showing an example of an arithmetic operation procedure of selecting an image in the arithmetic operation unit 100e according to the present embodiment.

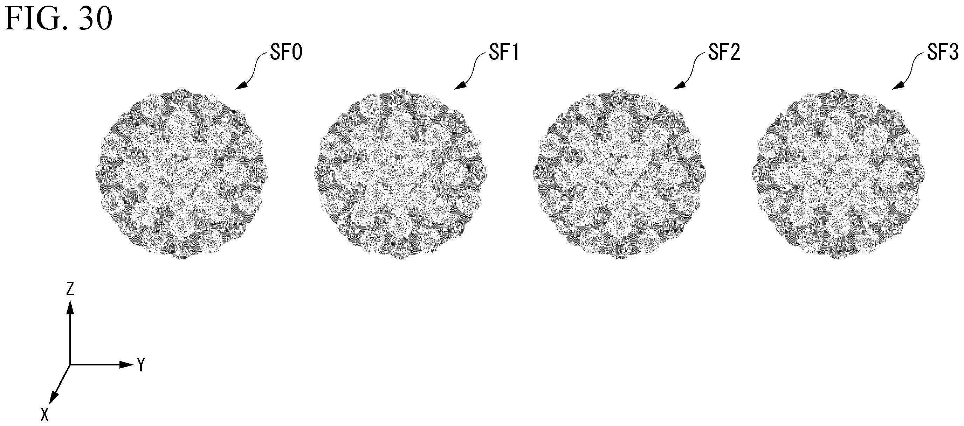

The analysis image acquisition unit 107e acquires a spheroid image SF0, a spheroid image SF1, a spheroid image SF2, and a spheroid image SF3, which are an analysis image group captured by the imaging unit 22 (step S1000). Here, the spheroid image will be described with reference to Fig. 30.

Fig. 30 is a diagram showing an example of a spheroid image according to the present embodiment. In Fig. 30, a three-dimensional image in which each spheroid is imaged is referred to as the spheroid image. The spheroid image SF0, the spheroid image SF1, the spheroid image SF2, and the spheroid image SF3 are three-dimensional images obtained by imaging a plurality of spheroids cultured under the same conditions. The spheroid image SF0, the spheroid image SF1, the spheroid image SF2, and the spheroid image SF3 are three-dimensional images in which spheroids that are not uniform in directions of the X axis, the Y axis, and the Z axis are imaged.

Returning to Fig. 29, a description of the process of the arithmetic operation unit 100e will be continued.

The analysis image acquisition unit 107e sets the spheroid image SF0 as a reference image group and supplies the reference image group to the reference image acquisition unit 101e. A user of the calculation device 10e may pre-designate a spheroid image to be set as the reference image group in the analysis image acquisition unit 107e among the spheroid images that are the analysis image group captured by the imaging unit 22.

The analysis image acquisition unit 107e supplies the comparative image acquisition unit 102e with the spheroid image SF1, the spheroid image SF2, and the spheroid image SF3 as a comparative image group.

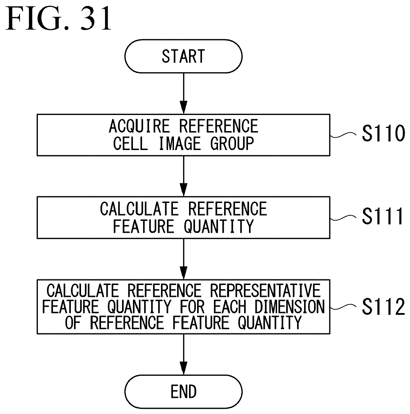

The reference image acquisition unit 101e and the comparative image difference calculation unit 103e execute a process of calculating the reference representative feature quantity from the reference image group (step S1001). Here, the process of calculating the reference representative feature quantity will be described with reference to Fig. 31.

Fig. 31 is a flowchart showing an example of an arithmetic operation procedure of calculating a reference representative feature quantity in the arithmetic operation unit 100e according to the present embodiment.

The reference image acquisition unit 101e acquires the spheroid image SF0 which is the reference image group supplied by the analysis image acquisition unit 107e (step S110). The reference image acquisition unit 101e supplies the acquired spheroid image SF0 to the reference feature quantity calculation unit 1031e.

The reference feature quantity calculation unit 1031e calculates a reference feature quantity from the spheroid image SF0 supplied by the reference image acquisition unit 101e (step S111). Here, the reference feature quantity calculation unit 1031e calculates a feature quantity tensor for each of voxels extracted from the spheroid image SF0 as the reference feature quantity. The reference feature quantity calculation unit 1031e supplies a plurality of feature quantity tensors that have been calculated to the representative feature quantity calculation unit 1033e.

The representative feature quantity calculation unit 1033e calculates a reference representative feature quantity tensor from the plurality of feature quantity tensors of the spheroid image SF0 supplied by the reference feature quantity calculation unit 1031e (step S112). The representative feature quantity calculation unit 1033e supplies the calculated reference representative feature quantity tensor to the distance calculation unit 1034e.

Returning to Fig. 29, a description of the process of the arithmetic operation unit 100e will be continued.

The comparative image acquisition unit 102e acquires a spheroid image SF1, a spheroid image SF2, and a spheroid image SF3 supplied by the analysis image acquisition unit 107e as a comparative image group (step S1002). The comparative image acquisition unit 102e supplies the spheroid image SF1, the spheroid image SF2, and the spheroid image SF3 that have been acquired to the comparative feature quantity calculation unit 1032e.

The comparative feature quantity calculation unit 1032e calculates feature quantity tensors of the spheroid images included in the spheroid image SF1, the spheroid image SF2, and the spheroid image SF3, which are the comparative image group supplied by the reference image acquisition unit 101e, as a plurality of comparative feature quantities (step S1003). The comparative feature quantity calculation unit 1032e supplies the plurality of comparative feature quantities that have been calculated to the distance calculation unit 1034e.

The representative feature quantity calculation unit 1033e calculates a comparative representative feature quantity tensor from the plurality of feature quantity tensors for each of the spheroid image SF1, the spheroid image SF2, and the spheroid image SF3 supplied by the comparative feature quantity calculation unit 1032e (step S1004). The representative feature quantity calculation unit 1033e supplies a plurality of comparative representative feature quantity tensors that have been calculated to the distance calculation unit 1034e.

The distance calculation unit 1034e calculates distances between the reference representative feature quantity tensor supplied by the representative feature quantity calculation unit 1033e and the plurality of comparative representative feature quantity tensors (step S1005). Here, the distance between the feature quantity tensors is, for example, a Euclidean distance calculated on the basis of the difference between values of components of the feature quantity tensors. Also, the distance between the feature quantity tensors may be a distance other than the Euclidean distance. The distance calculation unit 1034e supplies a plurality of distances that have been calculated to the image selection unit 104e.

The image selection unit 104e selects a spheroid image from the comparative image group on the basis of the plurality of distances supplied by the distance calculation unit 1034e (step S1006). Here, the image selection unit 104e determines a smallest distance from the plurality of distances supplied by the distance calculation unit 1034e. The image selection unit 104e selects a spheroid image that is a comparative image group corresponding to the determined smallest distance. The image selection unit 104e supplies information representing the selected spheroid image to the result output unit 300.

Also, the image selection unit 104e may determine a largest distance from the plurality of distances supplied by the distance calculation unit 1034e and select the spheroid image from the comparative image group.

On the basis of information representing the spheroid image supplied by the image selection unit 104e, the result output unit 300 causes the display unit 30 to display the spheroid image represented by the information (step S 1007).

In the above-described embodiment, the term "on the basis of" may be used instead of the term "using" in the description such as a "feature quantity calculated using a plurality of reference images." That is, in the case of this example, the description of a "feature quantity calculated using a plurality of reference images" may be referred to as a "feature quantity calculated on the basis of a plurality of reference images." That is, in the above-described embodiment, a description in which the terms "using" and "on the basis of" are replaced with each other is also included in the description of the embodiment.

Also, the various processes described above may be performed by recording a program for executing processes of the calculation device 10 according to the embodiment of the present invention on a computer-readable recording medium and causing a computer system to read and execute the program recorded on the recording medium.

Also, the "computer system" used here may include an operating system (OS) and hardware such as peripheral devices. Also, the "computer system" is assumed to include a homepage providing environment (or displaying environment) when a World Wide Web (WWW) system is used. Also, the "computer-readable recording medium" refers to a storage device such as a flexible disc, a magneto-optical disc, a read-only memory (ROM), a writable non-volatile memory such as a flash memory, a portable medium such as a compact disc-ROM (CD-ROM), and a hard disk embedded in the computer system.

Furthermore, the "computer-readable recording medium" is assumed to include a medium that holds a program for a constant period of time, such as a volatile memory (for example, a dynamic random access memory (DRAM)) inside a computer system serving as a server or a client when the program is transmitted via a network such as the Internet or a communication circuit such as a telephone circuit. Also, the above-described program may be transmitted from a computer system storing the program in a storage device or the like to another computer system via a transmission medium or by transmission waves in a transmission medium. Here, the "transmission medium" for transmitting the program refers to a medium having a function of transmitting information, such as a network (a communication network) like the Internet or a communication circuit (a communication line) like a telephone circuit. Also, the above-described program may be a program for implementing some of the above-described functions. Further, the above-described program may be a program capable of implementing the above-described function in combination with a program already recorded on the computer system, i.e., a so-called differential file (differential program).

Although embodiments of the present invention have been described with reference to the drawings, specific configurations are not limited to the embodiments and designs and the like may be made without departing from the scope of the present invention.

### [Reference Signs List]

1 Microscope observation system
10, 10a, 10b, 10c, 10d, 10e Calculation device
20 Microscope device
30 Display unit
100, 100a, 100b, 100c, 100e Arithmetic operation unit
101, 101b, 101e Reference image acquisition unit
101c Classification reference image acquisition unit
102, 102b, 102d, 102e Comparative image acquisition unit
102c Target image acquisition unit
103, 103b, 103c Calculation unit
103d, 103e Comparative image difference calculation unit
104 Selection unit
104d Abnormal image selection unit
104e Image selection unit
105 Proportion calculation unit
106, 106e Position determination unit
107, 107e Analysis image acquisition unit
108 Analysis unit
109 Classification unit
110 Culture state determination unit
1031, 1031b, 1031e Reference feature quantity calculation unit
1031c Classification reference feature quantity calculation unit
1032, 1032e Comparative feature quantity calculation unit
1033, 1033e Representative feature quantity calculation unit
1034, 1034d, 1034e Distance calculation unit
1081 Time-series calculation unit
1082 Concentration change calculation unit
1083 Type change calculation unit
200, 200c Storage unit
201 Dimension reduction information storage unit
202, 202b Reference image storage unit
202c Classification reference image storage unit
300 Result output unit

## Claims

1. A calculation device comprising:
a reference image acquisition unit configured to acquire a plurality of reference images in which cells are imaged;
a comparative image acquisition unit configured to acquire a comparative image in which comparative cells to be compared with the cells imaged in the plurality of reference images are imaged; and
a calculation unit configured to calculate a difference between a feature quantity calculated using the plurality of reference images and a feature quantity calculated using the comparative image.

2. The calculation device according to claim 1,
wherein the comparative image acquisition unit is configured to acquire a plurality of comparative images and
wherein the calculation unit is configured to calculate a difference between the feature quantity calculated using the plurality of reference images and a feature quantity calculated using the plurality of comparative images.

3. The calculation device according to claim 2, wherein the feature quantity calculated on the basis of the plurality of comparative images includes a plurality of feature quantities.

4. The calculation device according to any one of claims 1 to 3, wherein the feature quantity calculated on the basis of the plurality of reference images includes a plurality of feature quantities.

5. The calculation device according to any one of claims 1 to 4, wherein the calculation unit is configured to calculate the difference using the reference image acquired by the reference image acquisition unit, the comparative image acquired by the comparative image acquisition unit, and feature quantities obtained by dimensionally reducing the reference image and the comparative image.

6. The calculation device according to claim 5, wherein the calculation unit is configured to calculate the difference using a feature quantity after dimension reduction through a neural network including a plurality of layers of an input layer, one or more intermediate layers, and an output layer and configured to transfer information representing that a node of each layer has assigned a predetermined weight to each node of the next layer and subsequent layers.

7. The calculation device according to claim 6, wherein the calculation unit uses an output of any one of the intermediate layers constituting the neural network.

8. The calculation device according to any one of claims 1 to 7, wherein the difference calculated by the calculation unit is a value calculated on the basis of a relationship between corresponding values among one or more values representing the feature quantity calculated using the plurality of reference images and one or more values representing the feature quantity calculated using the comparative image.

9. The calculation device according to any one of claims 1 to 8, comprising an image selection unit configured to select the comparative image in which the difference calculated by the calculation unit is larger than a predetermined value.

10. The calculation device according to claim 9, comprising a proportion calculation unit configured to calculate a proportion of comparative images in which the difference calculated by the calculation unit is larger than the predetermined value from a plurality of comparative images using a selection result of the image selection unit.

11. The calculation device according to claim 9 or 10,
wherein each of a plurality of comparative images corresponds to one of a plurality of positions of a culture vessel where the cells are cultured and
wherein the calculation device comprises a position determination unit configured to determine a position corresponding to the comparative image in which the difference calculated by the calculation unit is larger than the predetermined value using a selection result of the image selection unit.

12. The calculation device according to any one of claims 1 to 11,
wherein the comparative image is an image captured in time series and
wherein the calculation device comprises a time-series calculation unit configured to calculate the difference for each time in the time series using the difference calculated by the calculation unit.

13. The calculation device according to any one of claims 1 to 12,
wherein the comparative image is an image of the cells imaged for each concentration of a compound added to the comparative cells and
wherein the calculation device comprises a concentration change calculation unit configured to calculate the difference for each concentration using the difference calculated by the calculation unit.

14. The calculation device according to any one of claims 1 to 13,
wherein the comparative image is an image of the cells imaged for each type of compound added to the comparative cells and
wherein the calculation device comprises a type change calculation unit configured to calculate the difference for each type using the difference calculated by the calculation unit.

15. The calculation device according to any one of claims 1 to 14,
wherein the reference image acquisition unit is configured to acquire a plurality of types of reference images,
wherein the calculation unit is configured to calculate differences between the plurality of types of reference images and the comparative image, and
wherein the calculation unit comprises a classification unit configured to classify the comparative image using a plurality of differences calculated by the calculation unit.

16. The calculation device according to any one of claims 1 to 15, comprising:
a comparative image difference calculation unit configured to calculate a difference between comparative images; and
an abnormal image selection unit configured to select the comparative image for which the difference between the comparative images calculated by the comparative image difference calculation unit among the comparative images is larger than a predetermined value as an abnormal image.

17. The calculation device according to any one of claims 1 to 16, comprising a culture state determination unit configured to determine a culture state of the comparative cells imaged in the comparative image on the basis of whether or not the difference between the comparative images is within a predetermined range.

18. The calculation device according to any one of claims 1 to 17, wherein the feature quantity includes an image feature quantity regarding the cells.

19. A calculation program for causing a computer to execute:
a reference image acquisition step of acquiring a plurality of reference images in which cells are imaged;
a comparative image acquisition step of acquiring a comparative image in which comparative cells to be compared with the cells imaged in the plurality of reference images are imaged; and
a calculation step of calculating a difference between a feature quantity calculated using the plurality of reference images and a feature quantity calculated using the comparative image.

20. A calculation method for executing:
a reference image acquisition means for acquiring a plurality of reference images in which cells are imaged;
a comparative image acquisition means for acquiring a comparative image in which comparative cells to be compared with the cells imaged in the plurality of reference images are imaged; and
a calculation means for calculating a difference between a feature quantity calculated using the plurality of reference images and a feature quantity calculated using the comparative image.
